# EUROPEAN PATENT APPLICATION

(11) **EP 4 610 657 A1**
(43) Date of publication of application: **03.09.2025**
(21) Application number: 23880904.0
(22) Date of filing: 27.10.2023
(51) Int. Cl.: G01N 33/68, A61K 45/00, A61K 51/08, A61P 29/00

(54) **DIAGNOSIS OF IMMUNE-MEDIATED INFLAMMATORY DISEASES USING MMP12 AS INDICATOR, AND MEDICINE FOR TREATING IMMUNE-MEDIATED INFLAMMATORY DISEASES VIA MMP12 INHIBITION**

(30) Priority: 28.10.2022 JP 2022173336
(71) Applicant: Keio University, Tokyo, 108-8345 (JP)
(72) Inventor: MATSUMOTO, Kotaro, Tokyo 160-8582 (JP); KANEKO, Yuko, Tokyo 160-8582 (JP); SUZUKI, Katsuya, Tokyo 160-8582 (JP); HANAOKA, Kenjiro, Tokyo 105-8512 (JP); SASAKI, Eita, Tokyo 105-8512 (JP); TAKESHITA, Masaru, Tokyo 160-8582 (JP); YAMADA, Sota, Tokyo 105-8512 (JP); MARUOKA, Ryuta, Tokyo 105-8512 (JP)
(74) Representative: Wächtershäuser & Hartz Patentanwaltspartnerschaft mbB
(86) International application number: PCT/JP2023/038959
(87) International publication number: WO 2024/090571

(57) **Abstract**

Provided are a method for detecting an immune-mediated inflammatory disease characterized by an increase in expression of MMP12, in a subject, a diagnostic drug containing a substance that specifically interacts with MMP12, and a therapeutic agent containing an MMP12 inhibitory substance.

## Description

### Technical Field

The present invention relates to a method for detecting an immune-mediated inflammatory disease, a biomarker for diagnosing an immune-mediated inflammatory disease, a diagnostic drug for an immune-mediated inflammatory disease, a therapeutic agent for an immune-mediated inflammatory disease, and a diagnostic kit.

### Background Art

Granulomatous vasculitis is a type of immune-mediated inflammatory disease, and Takayasu arteritis (TAK), giant cell arteritis (GCA), and eosinophilic granulomatosis with polyangiitis (EGPA), granulomatosis with polyangiitis (GPA), and microscopic polyangiitis (MPA), which are classified into ANCA (anti-neutrophil cytoplasmic antibody)-associated vasculitis, are all designated intractable diseases. According to statistics for 2020 from the Japan Intractable Diseases Research Foundation/Intractable Diseases Information Center, the number of people who have received a recipient certificate was approximately 4700 for patients with Takayasu arteritis, about 1700 for patients with giant cell arteritis, about 5200 for patients with eosinophilic granulomatosis with polyangiitis, about 3200 for patients with granulomatosis with polyangiitis, and about 11000 for patients with microscopic polyangiitis.

In these granulomatous vasculitides, the chronic granulomatous inflammation of the vessel wall causes obstruction and/or rupture of the blood vessels, and the prognosis is generally poor. The disease condition is evaluated by diagnostic imaging and an increase in serum CRP.

Treatment includes immunosuppressive treatment with steroids (prednisolone (registered trademark)), cyclophosphamide (Endoxan (registered trademark)), methotrexate (Rheumatrex (registered trademark)), as well as rituximab (Rituxan (registered trademark)), which is B-cell targeted treatment, avacopan (TAVNEOS (registered trademark)), which is a complement C5a selective inhibitor, and tocilizumab (Actemra (registered trademark)), which is an interleukin-6 (IL-6) inhibitor therapy (Non-Patent Literatures 1 and 2), but long-term administration causes large blood vessel stenosis and dilation to progress without symptoms.

### Citation List

### Non-Patent Literature

NPTL 1: N Engl J Med 2017; 377:317-328
NPTL 2: Ann Rheum Dis. 2018 Mar;77(3):348-354

### Summary of Invention

### Technical Problem

The problem to be solved by the present invention is to provide a method for detecting an immune-mediated inflammatory disease characterized by an increase in expression of MMP12, a biomarker for diagnosing the immune-mediated inflammatory disease, a diagnostic drug for the immune-mediated inflammatory disease, a diagnostic kit for the immune-mediated inflammatory disease, and a therapeutic agent for the immune-mediated inflammatory disease, which do not use IL-6 as an indicator.

### Solution to Problem

The present invention includes embodiments described below.

Item 1. A method for detecting an immune-mediated inflammatory disease characterized by an increase in expression of MMP12, in a subject, the method including
measuring a level of an MMP12 protein in a biological sample obtained from the subject.

Item 2. The method according to Item 1, further including bringing the biological sample obtained from the subject into contact with a substance that binds to MMP12, wherein
the measuring the level of the MMP12 protein includes measuring a level of a complex of the MMP12 protein and a substance that binds to the MMP12 protein.

Item 3. The method according to Item 1, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 4. The method according to Item 1, wherein the biological sample is blood, serum, or plasma.

Item 5. The method according to Item 1, wherein the immune-mediated inflammatory disease characterized by an increase in expression of MMP12 is Takayasu arteritis.

Item 6. The method according to Item 1, further including bringing the biological sample obtained from the subject into contact with a peptide having a site that can be cleaved by MMP12, wherein
the measuring the level of the MMP12 protein includes measuring a change caused by cleavage of the peptide having a site that can be cleaved by MMP12.

Item 7. The method according to any one of Items 1 to 5, wherein
the method is a method for predicting a possibility of developing the immune-mediated inflammatory disease characterized by an increase in expression of MMP12, in a subject,
the method further includes comparing the level of the MMP12 protein in the biological sample obtained from the subject with a reference value, and
if the level of the MMP12 protein in the biological sample obtained from the subject is higher than the reference value, it indicates that there is a high possibility that the subject will develop the immune-mediated inflammatory disease.

Item 8. The method according to any one of Items 1 to 5, wherein
the method is a method for predicting prognosis of a subject with the immune-mediated inflammatory disease characterized by an increase in expression of MMP12,
the subject is a subject who has received treatment for the immune-mediated inflammatory disease characterized by an increase in expression of MMP12,
the method further includes comparing the level of the MMP12 protein in the biological sample obtained from the subject with a reference value, and
if the level of the MMP12 protein in the biological sample obtained from the subject is higher than the reference value, it indicates that there is a high possibility of relapse of the immune-mediated inflammatory disease characterized by an increase in expression of MMP12, in the subject.

Item 9. The method according to any one of Items 1 to 5, wherein
the method is a method for predicting a post-treatment condition of a subject with the immune-mediated inflammatory disease characterized by an increase in expression of MMP12,
the subject is a subject who has received treatment for the immune-mediated inflammatory disease characterized by an increase in expression of MMP12,
the method further includes comparing the level of the MMP12 protein in the biological sample obtained from the subject with a reference value, and
if the level of the MMP12 protein in the biological sample obtained from the subject is lower than the reference value, it indicates that there is a high possibility that the immune-mediated inflammatory disease characterized by an increase in expression of MMP12, in the subject, has remitted.

Item 10. The method according to any one of Items 1 to 5, wherein
the method is a method for evaluating timing of administration of a therapeutic drug for the immune-mediated inflammatory disease characterized by an increase in expression of MMP12, to a subject with the immune-mediated inflammatory disease characterized by an increase in expression of MMP12,
the method further includes comparing the level of the MMP12 protein in the biological sample obtained from the subject with a reference value, and
a fact that the level of the MMP12 protein in the biological sample obtained from the subject is lower than the reference value is an indicator for discontinuing the administration of the drug to the subject.

Item 11. A diagnostic biomarker for an immune-mediated inflammatory disease characterized by an increase in expression of MMP12, including MMP12.

Item 12. A diagnostic drug for an immune-mediated inflammatory disease characterized by an increase in expression of MMP12, containing a substance that specifically interacts with MMP12.

Item 13. The diagnostic drug according to Item 12, wherein the diagnostic drug is a diagnostic drug including a conjugate of a peptide having a site that can be cleaved by MMP12 and a label substance.

Item 14. The diagnostic drug according to Item 12, wherein the conjugate includes a conjugate including a peptide having a site that can be cleaved by MMP12, a fluorophore bound to one end of the peptide, and a quencher bound to another end of the peptide.

Item 15. The diagnostic drug according to any one of Items 12 to 14, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 16. A diagnostic kit for diagnosing an immune-mediated inflammatory disease characterized by an increase in expression of MMP12, the diagnostic kit including a substance that specifically interacts with an MMP12 protein.

Item 17. The diagnostic kit according to Item 16, wherein the substance that specifically interacts with the MMP12 protein includes an antibody to MMP12, an antigen-binding fragment of an antibody to MMP12, an aptamer to MMP12, or a peptide having a site that can be cleaved by MMP12.

Item 18. A method for screening for a substance effective for treatment for an immune-mediated inflammatory disease characterized by an increase in expression of MMP12, the method including:
measuring a level of an MMP12 protein in a biological sample obtained from a subject after administration of a test substance; and
if the level of the MMP12 protein is decreased due to the administration of the test substance, selecting the test substance as a candidate substance effective for treatment for the immune-mediated inflammatory disease characterized by an increase in expression of MMP12.

Item 19. A therapeutic agent for an immune-mediated inflammatory disease characterized by an increase in expression of MMP12, containing an MMP12 inhibitory substance.

### Advantageous Effects of Invention

With the method for detecting an immune-mediated inflammatory disease characterized by an increase in expression of MMP12, the diagnostic drug, and the diagnostic kit according to the present invention, it is possible to detect or diagnose this disease with the same or higher accuracy than conventional prediction methods, using MMP12. The diagnostic drug and the diagnostic kit do not use IL-6 as an indicator and can be used even under IL-6 inhibitory treatment.

In addition, with the method for screening for a substance effective for treatment for an immune-mediated inflammatory disease characterized by an increase in expression of MMP12 according to the present invention, it is possible to effectively screen for a therapeutic agent for this disease.

In addition, with the therapeutic agent for an immune-mediated inflammatory disease characterized by an increase in expression of MMP12 according to the present invention, it is possible to treat this disease.

### Brief Description of Drawings

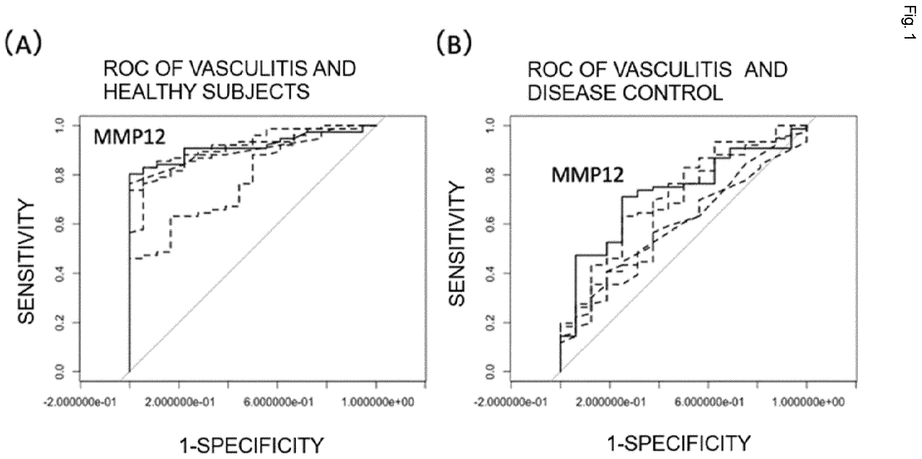

Fig. 1 shows graphs of ROC analysis, wherein (A) shows comparison of a group of patients with vasculitis syndrome and a group of healthy subjects and (B) shows comparison of the group of patients with vasculitis syndrome and groups of patients with immune-mediated inflammatory diseases other than vasculitis syndrome.

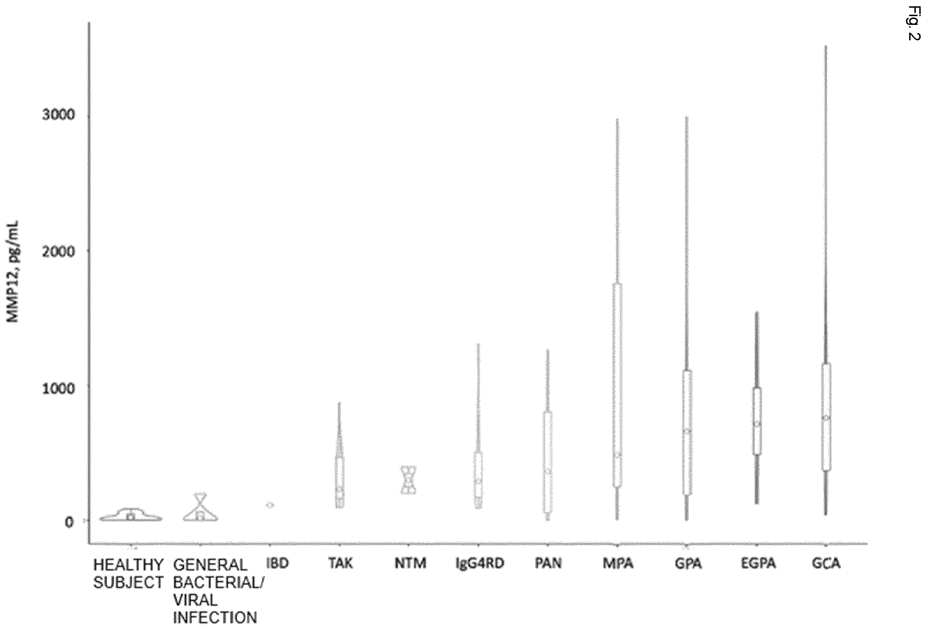

Fig. 2 is a graph showing the value of serum MMP12 of each patient group, wherein IBD: inflammatory bowel disease, TAK: Takayasu arteritis, NTM: nontuberculous mycobacterial infection, IgG4RD: IgG4 related disease, PAN: polyarteritis nodosa, MPA: microscopic polyangiitis, GPA: granulomatosis with polyangiitis, EGPA: eosinophilic granulomatosis with polyangiitis, and GCA: giant cell arteritis.

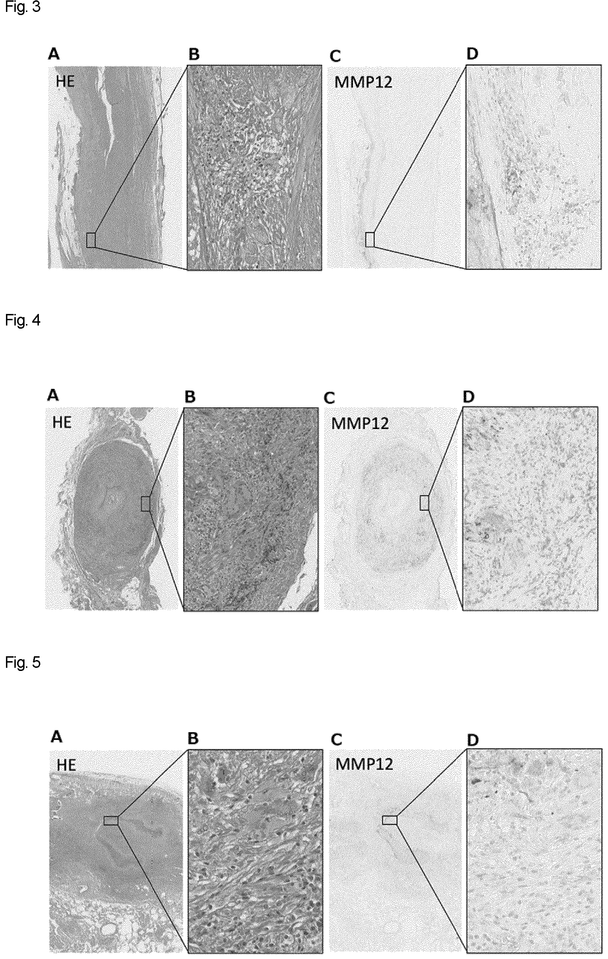

Fig. 3 shows expression of MMP12 in the aorta of a patient with Takayasu arteritis, wherein (A) shows HE staining, (B) is an enlarged picture of a portion surrounded by a rectangle in Fig. 3(A), (C) shows immunohistochemical staining of MMP12, and (D) is an enlarged picture of a portion surrounded by a rectangle in Fig. 3(C).
Fig. 4 shows expression of MMP12 in the temporal artery of a patient with giant cell arteritis, wherein (A) shows HE staining, (B) is an enlarged picture of a portion surrounded by a rectangle in Fig. 4(A), (C) shows immunohistochemical staining of MMP12, and (D) is an enlarged picture of a portion surrounded by a rectangle in Fig. 4(C).
Fig. 5 shows expression of MMP12 in the lung of a patient with polyangiitis granulomatosis, wherein (A) shows HE staining, (B) is an enlarged picture of a portion surrounded by a rectangle in Fig. 5(A), (C) shows immunohistochemical staining of MMP12, and (D) is an enlarged picture of a portion surrounded by a rectangle in Fig. 5(C).

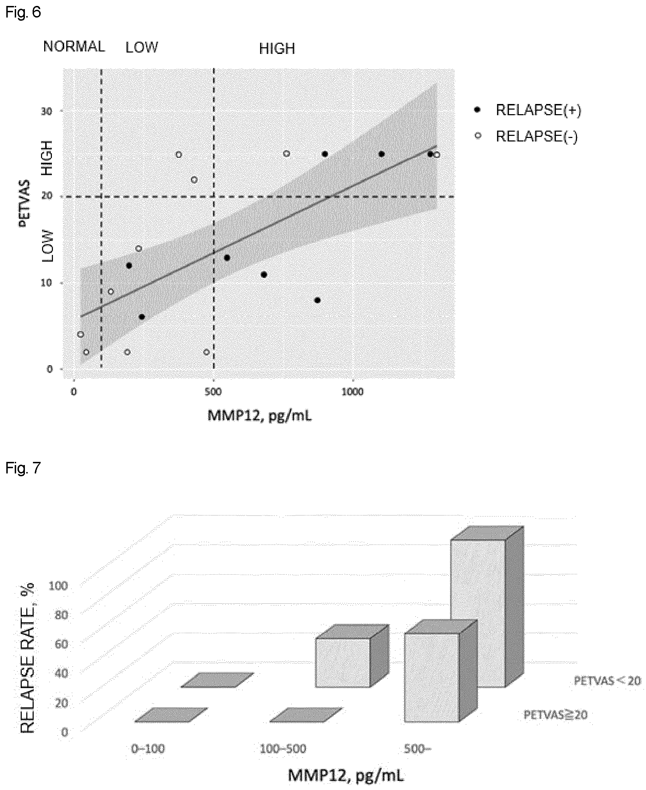

Fig. 6 is a graph showing the correlation between PETVAS and MMP12 at the time of diagnosis of Takayasu arteritis and giant cell arteritis.
Fig. 7 is a graph showing the relationship between the value of PETVAS and the level of serum MMP12, and a relapse rate.

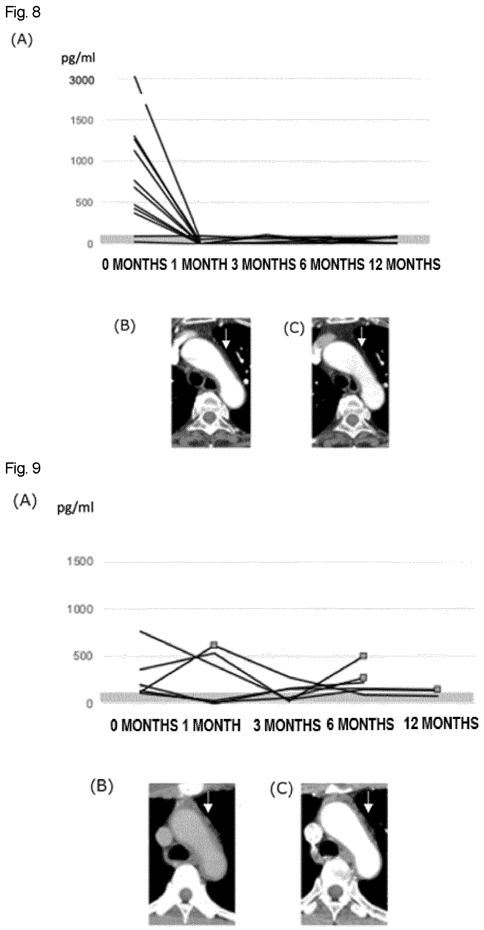

Fig. 8(A) shows changes in the value of MMP12 over time in monotonous descending type patients before and after treatment, Fig. 8(B) is an image of the vessel wall of one monotonous descending type male patient with Takayasu arteritis before treatment, and Fig. 8(C) is an image of the vessel wall of the same patient as in Fig. 8(B) 8 months after the start of treatment.
Fig. 9(A) shows changes in the value of MMP12 over time in monotonous smoldering type patients before and after treatment, Fig. 9(B) is an image of the vessel wall of one smoldering type male patient with Takayasu arteritis before treatment, and Fig. 9(C) is an image of the vessel wall of the same patient as in Fig. 9(B) 6 months after the start of treatment.

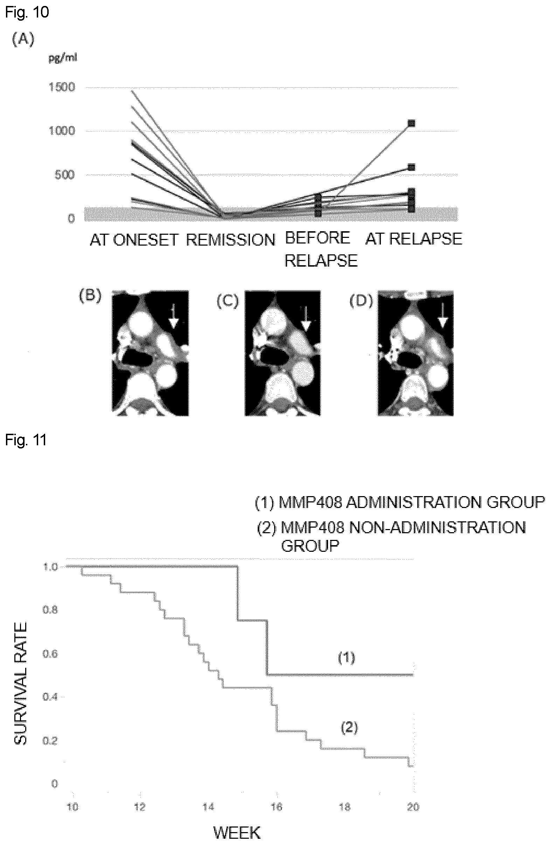

Fig. 10(A) shows changes in the value of MMP12 over time in patients with relapse type Takayasu arteritis before and after treatment, Fig. 10(B) is an image of the vessel wall of one male patient with Takayasu arteritis, Fig. 10(C) is an image of the vessel wall of the same patient as in Fig. 10(B) during remission 1 month after the start of treatment, and Fig. 10(D) is an image of the vessel wall of the same patient as in Fig. 10(B) at the time of relapse 4 months after Fig. 10(C).
Fig. 11 shows survival curves for mice in a non-MMP administration group and an MMP administration group.

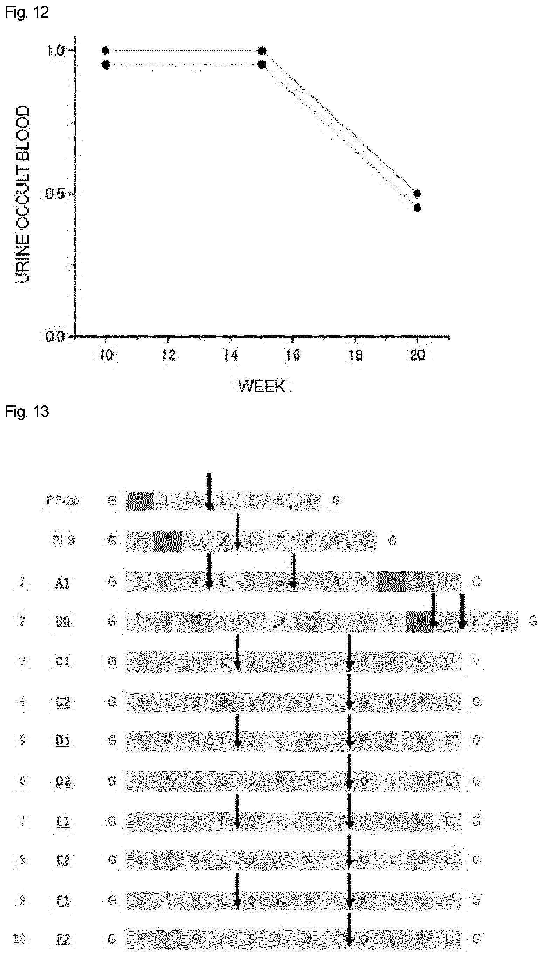

Fig. 12 shows urine occult blood of mice in the MMP administration group at weeks 10, 15, and 20 with n=2.
Fig. 13 shows sequences and cleavage sites of two positive controls and candidate peptide Nos. 1 to 10.

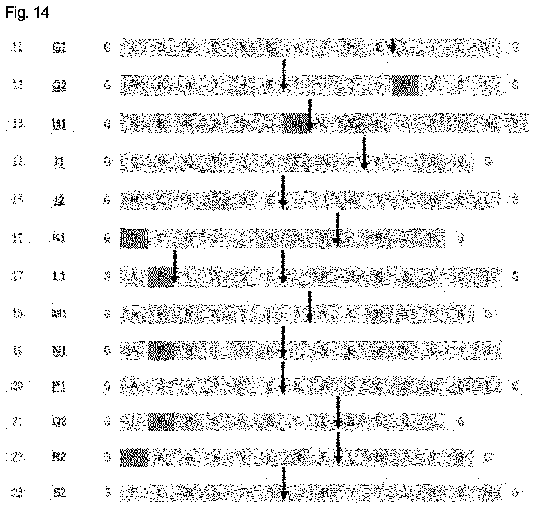

Fig. 14 shows sequences and cleavage sites of candidate peptide Nos. 11 to 23.

### Description of Embodiments

In this description, a singular form includes a singular number and a plural number except for the case where an explicit description is separately made in this description or the case where a clear contradiction occurs in terms of context.

In this description, "containing" and "including" are also concepts that include "consisting only of'.

In numerical ranges described herein in stages, the upper limit value or lower limit value of a numerical range at a certain stage may be arbitrarily combined with the upper limit value or lower limit value of a numerical range at any other stage. In addition, in a numerical range described herein, the upper limit value or lower limit value of the numerical range may be replaced with a value described in Examples or a value that may be uniquely derived from Examples. Furthermore, in this description, numerical values connected by "to" mean a numerical range including the numerical values before and after "to" as a lower limit value and an upper limit value.

As used herein, the term "MMP12" refers to matrix metalloproteinase 12 protein. Human MMP12 is a protein consisting of 470 amino acids, represented by NCBI Reference Sequence: NP#002417 (Uniprot P39900), and mouse MMP12 is a protein consisting of 473 amino acids, represented by NCBI Reference Sequence NP#032631 (Uniprot 34690). MMP12 is sometimes written as MMP-12, and these terms can be used interchangeably.

As used herein, the "immune-mediated inflammatory disease characterized by an increase in expression of MMP12" includes vasculitis syndrome, granulomatous vasculitis, IgG4 related disease, inflammatory bowel disease (including Crohn's disease and ulcerative colitis), sarcoidosis, tuberculous mycobacterial infection, nontuberculous mycobacterial infection. Vasculitis is a general term for diseases in which inflammation is observed in the blood vessels themselves, and is also called vasculitis syndrome. Vasculitis syndrome is classified into three categories according to the size of the blood vessels: large-vessel vasculitis, medium-vessel vasculitis, and small-vessel vasculitis. Large-vessel vasculitis includes Takayasu arteritis (TAK) and giant cell arteritis (GCA). Medium-vessel vasculitis includes polyarteritis nodosa and Kawasaki disease. Small-vessel vasculitis includes eosinophilic granulomatosis with polyangiitis (EGPA), granulomatosis with polyangiitis (GPA), and microscopic polyangiitis (MPA), which are classified as ANCA (anti-neutrophil cytoplasmic antibody)-associated vasculitis.

As used herein, the "detection" of the immune-mediated inflammatory disease characterized by an increase in expression of MMP12 includes determining this disease, determining which patients (responders) will respond to a therapeutic agent (companion diagnosis method), determining a preventive effect for this disease, determining a therapeutic effect for this disease, tests for assisting in the diagnosis of this disease, and tests for treatment (particularly early treatment) for this disease.

As used herein, the "determination" of the immune-mediated inflammatory disease characterized by an increase in expression of MMP12 includes not only determining whether or not this disease has occurred, but also prophylactically determining the possibility of developing this disease, predicting the prognosis of this disease after treatment, determining the possibility of remission, determining the possibility of relapse, evaluating the timing of discontinuation of drug administration, and determining the therapeutic effect of a therapeutic agent for this disease.

As used herein, the term "increase in expression of MMP12" in the "immune-mediated inflammatory disease characterized by an increase in expression of MMP12" refers to an increase in the level of MMP12 in a biological sample obtained from a subject, as compared to the level of MMP12 in the subject before the onset of the immune-mediated inflammatory disease or the average of the levels of MMP12 in healthy subjects.

As used herein, the term "immune-mediated inflammatory disease" refers to a chronic inflammatory disease in which the immune system causes damage to tissues through an immune response (e.g., a B-cell response or a T-cell response) to an antigen (i.e., an autoantigen) that is a part of a normal host or without an immune response to the antigen.

As used herein, the term "treatment" means cure or improvement of a disease or symptom, or suppression of a symptom, and includes "prevention". The term "prevention" means prevention of the onset of a disease or symptom.

As used herein, the term "subject" refers to a mammal including human, mouse, rat, cow, horse, pig, monkey, dog, cat, rabbit, goat, or sheep, preferably human or mouse, and more preferably human.

As used herein, the term "biological sample" may be a body fluid, tissue, or cell. Examples of body fluids include blood (e.g., whole blood), blood cells, serum, plasma, pleural fluid, ascitic fluid, cerebrospinal fluid, saliva, urine, and feces Examples of tissues include heart, artery, kidney, lung, inner ear, paranasal sinus, skin, nerves, and blood vessels of other organs. The biological sample may be obtained from the patient and used as is, or may be subjected to pretreatment such as filtration, distillation, extraction, concentration, centrifugation, inactivation of interfering components, and addition of reagents, in order to modify the characteristics of the sample, by other methods known in the art.

As used herein, the "level" of MMP12 can be the amount (e.g., expression amount) or concentration of the MMP12 protein.

The inventors identified a plurality of molecules specific to vasculitis through cross-disease serum proteome analysis. When the identified molecules were compared between a group of patients with vasculitis syndrome and a group of healthy subjects, and between a group of patients with vasculitis syndrome and a group of patients with immune-mediated inflammatory diseases other than vasculitis syndrome, it was found that MMP12 had the highest sensitivity for ROC curve determination. MMP12 was normal in patients with general bacterial and viral infections, so that it was also possible to use MMP12 to distinguish between these infections and vasculitis syndrome.

Next, the expression of MMP12 in the vasculitis tissues of Takayasu arteritis (TAK), giant cell arteritis (GCA), and granulomatosis with polyangiitis (GPA), which are immune-mediated inflammatory diseases characterized by an increase in expression of MMP12, was examined. The expression of MMP12 in the aortae of patients with Takayasu arteritis, in the temporal arteries of patients with giant cell arteritis, and in the lungs of patients with polyangiitis granulomatosis was confirmed, and the expression of MMP12 in the vasculitis tissues was confirmed.

As described above, the inventors found that the expression of MMP12 is associated with the onset of some immune-mediated inflammatory diseases characterized by an increase in expression of MMP12 and can be used as a diagnostic marker for immune-mediated inflammatory diseases characterized by an increase in expression of MMP12, and also found a diagnostic drug for immune-mediated inflammatory diseases characterized by an increase in expression of MMP12, a method for screening for a substance effective for treatment for immune-mediated inflammatory diseases characterized by an increase in expression of MMP12, and a therapeutic agent for immune-mediated inflammatory diseases characterized by an increase in expression of MMP12.

According to a first embodiment of the present invention, a method for detecting an immune-mediated inflammatory disease characterized by an increase in expression of MMP12, in a subject, including measuring a level of MMP12 in a biological sample obtained from the subject, is provided.

With this method, an examiner can easily and accurately detect the immune-mediated inflammatory disease, based on a test result in vitro. In addition, this method does not include a diagnosis method for a human, which is a medical practice.

The method for measuring the level of MMP12 in the biological sample obtained from the subject is not particularly limited, and the level of MMP12 can be measured using a substance that specifically interacts with MMP12. Examples of the substance that specifically interacts with MMP12 include substances that bind to MMP12 or substances that are cleaved by MMP12 due to the protease activity of MMP12.

In some embodiments, the level of MMP12 in the biological sample obtained from the subject may be measured by known protein identification methods, including but not limited to immunological measurement methods and mass spectrometry.

Examples of immunological measurement methods include, but are not limited to, enzyme antibody techniques (for example, ELISA), fluorescence antibody techniques, radioimmunoassay, solid-phase methods, sandwich methods and the like. For example, a substance that binds to MMP12 is bound to MMP to form a complex, and a signal obtained in accordance with the existing amount of the complex is directly or indirectly detected.

In some embodiments, the above method further includes bringing the biological sample obtained from the subject into contact with a substance that binds to MMP12, and the measuring the level of MMP12 includes measuring the level of a complex of MMP12 and the substance that binds to MMP12. The substance that binds to MMP12 is preferably a substance that specifically binds to MMP12. The term "specifically binds" means binding that is different from a non-specific interaction to a measurable extent, and refers to binding to each other.

In some embodiments, the substance that binds to MMP12 can be selected from the group consisting of an antibody to MMP12, an antigen-binding fragment of an antibody to MMP12, and an aptamer to MMP12.

As a label for detection or as an imaging probe, a radioactive nuclide, an enzyme, a fluorescent dye, and a contrast agent (e.g., paramagnetic ion) may be further bound to MM P12 and/or the substance that binds to MMP12.

An antibody to MMP12, that is, an anti-MMP12 antibody, can be produced using a known method for producing an antibody.

The method for producing an antibody is known (see, for example, Antibodies: A Laboratory Manual (1988) by Ed Harlow and David Lane, Cold Spring Harbor Laboratory Press, Cold Spring Harbor, New York). Examples of the antibody include polyclonal antibodies, monoclonal antibodies, chimeric antibodies and humanized antibodies, which are modified antibodies, and the like.

For example, antibody production can be induced by immunizing a host animal with the full-length polypeptide of MMP12 or a fragment thereof as an immunogen, that is, an epitope. The region of the polypeptide that is used as an immunogen when using an MMP12 fragment can be arbitrarily selected, or there are many publicized preparation examples of anti-MMP12 antibodies, including commercially available products, and thus it is also possible to refer to these publicized examples. The polypeptide to be used as an immunogen may be produced by a known standard method and can be chemically synthesized or biochemically synthesized as a recombinant protein. The polypeptide can also be obtained by using a commercially available custom synthesis service.

When producing an anti-MMP12 polyclonal antibody, the polypeptide to be used as an immunogen may immunize itself, or may immunize a recombinant cell or a reconstituted membrane that presents thereon a recombinant protein containing the polypeptide. The host animal to be immunized is not particularly limited and is preferably an animal species such as mouse, rat, rabbit, guinea pig, sheep, goat, donkey, chicken, or camel, more preferably mouse or rat, and particularly preferably mouse. Antiserum containing an anti-MMP12 antibody can be prepared by a known standard method. The antibody may be any class of the five immunoglobulin molecules (IgG, IgM, IgA, IgD, IgE) and is preferably IgG or IgM and more preferably IgG.

An anti-MMP12 monoclonal antibody can be prepared by fusing antibody-producing cells obtained in the preparation process described above with myeloma cells and then cloning the resulting cells. Alternatively, an anti-MMP12 monoclonal antibody can be prepared by expressing chemically-synthesized antibody genes in E. coli or other bacteria using genetic engineering techniques. A method for fusing antibody-producing cells with myeloma cells, a method for screening for desired cells from a cell group including fused cells, a method for monoclonalizing the cells selected by screening, and a method for preparing monoclonal antibodies from the clones can each be carried out by a known standard method. The synthesis of the desired monoclonal antibodies based on sequence information can also be carried out by a known standard method.

The MMP12 monoclonal antibody can be a genetically modified antibody that has been artificially modified for the purpose of reducing its xenogenicity to humans, for example. Examples of such antibodies include chimeric antibodies and humanized antibodies. These modified antibodies can be produced by known methods.

A chimeric antibody can be prepared by linking DNA encoding the variable (V) region of the anti-MM P12 monoclonal antibody of the present embodiment with DNA encoding the constant (C) region of a human antibody, incorporating the resulting DNA into an expression vector, and introducing the expression vector into a host.

A humanized antibody is obtained by grafting (CDR grafting) the CDRs of a non-human mammal antibody such as a mouse antibody onto the CDRs of a human antibody. The preparation of this antibody can be carried out as appropriate by applying general genetic recombination techniques. For example, a humanized antibody may be synthesized by PCR using a DNA sequence designed to encode an amino acid sequence that links each CDR of a mouse anti-MMP12 monoclonal antibody to the framework region of a human antibody, with a plurality of oligonucleotides produced so as to have overlapping portions at the terminal regions of both CDR and FR, as primers.

The FR of the variable region of a human antibody can be obtained from a publicized DNA database or the like. As the constant regions of the chimeric antibody and the humanized antibody, the constant region of a human antibody can be used. For example, Cγ1, Cγ2, Cγ3, and Cγ4 may be used for the heavy chain, and Cκ and Cλ may be used for the light chain.

An antigen-binding fragment of an antibody means a fragment including an antigen-binding portion of an antibody molecule. The substance that binds to MMP12 does not necessarily have to maintain the structure of the entire immunoglobulin molecule, if the substance can exhibit sufficient specificity and affinity in order to give specific antigen-binding properties. Since the antigen-binding ability of an antibody is dominated by the variable region of the antibody, the constant region portion of the immunoglobulin molecule does not necessarily have to be present. Therefore, examples of the antigen-binding fragment of the antibody of the present embodiment include Fab, Fab', F(ab')2, and Fd obtained by removing VL from Fab, each of which is a fragment composed of the variable portion of the immunoglobulin molecule, a single-chain Fv fragment (scFv), and a bispecific antibody (diabody) which is a dimer thereof.

An aptamer is an oligonucleotide that binds to its target with high affinity and specificity. An aptamer is a class of molecule that can replace an antibody in molecular recognition, and the aptamer to MMP12 is an oligonucleotide that has the ability to recognize MMP12 with high affinity and specificity. As is known to those skilled in the art, the aptamer can be obtained, as a substance that specifically binds to a target compound, by a known method in which a series of processes including "selection and amplification" is repeated in vitro, such as a method called a SELEX (Systematic Evolution of Ligands by EXponential enrichment) method (see, for example, JP 2006-320289A1, JP 2009-207491 A1, or JP 2009-165394 A1).

As the target compound for a nucleic acid aptamer as the substance that binds to MMP12, the full-length polypeptide of MMP12, typically, human MMP12, or a fragment thereof can be adopted and selected. The nucleic acid aptamer used in the present embodiment may have any binding activity as long as the nucleic acid aptamer specifically binds to MMP12, which is a target compound, and functions as an MMP12 inhibitory substance due to this binding, but the nucleic acid aptamer has a binding ability that exhibits a dissociation constant (KD) of 2 µM or less and preferably 600 nM or less for human MMP12.

Here, in this description, the dissociation constant (KD) refers to a constant obtained from the ratio of Kd to Ka (i.e., Kd/Ka) and is represented as a molar concentration (M). The KD value of the nucleic acid aptamer can be determined using a method established in the art. In a preferred method for determining the KD of the nucleic acid aptamer, surface plasmon resonance is used, and preferably, a biosensor system such as a Biacore (registered trademark) system is used.

In some embodiments, the above method further includes bringing the biological sample obtained from the subject into contact with a substance that is cleaved by MMP12, and the measuring the level of MMP12 includes measuring a change (e.g., fluorescence) caused by cleavage of the substance that is cleaved by MMP12. Examples of the substance that binds to MMP12 include peptides having a site that can be cleaved by MMP12. A diagnostic drug of a third embodiment described later can be used as the substance that is cleaved by MMP12.

In some embodiments, the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

In some embodiments, the immune-mediated inflammatory disease characterized by an increase in expression of MMP12 is granulomatous vasculitis.

In some embodiments, the biological sample is blood, serum, or plasma.

The method for detecting the immune-mediated inflammatory disease characterized by an increase in expression of MMP12 according to the first embodiment can be used for various tests or determinations.

In some embodiments, the above method is a method for predicting the possibility of developing the immune-mediated inflammatory disease characterized by an increase in expression of MMP12, in a subject, and the above method further includes comparing the level of MMP12 in the biological sample obtained from the subject with a reference value. If the level of MMP12 in the biological sample obtained from the subject is higher than the reference value, it indicates that there is a high possibility that the subject will develop the immune-mediated inflammatory disease characterized by an increase in expression of MMP12.

The above reference value is, for example, an average or median value calculated from measurement of the levels of MMP12 in a plurality of healthy subjects, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

If it is determined based on such a determination criterion that there is a high possibility that the subject will develop the immune-mediated inflammatory disease characterized by an increase in expression of MMP12, this determination result can be taken into account in determining a future treatment plan for the subject such as making an additional diagnosis of immune-mediated inflammatory disease. The method of the present embodiment is useful as a means for diagnosis (especially early diagnosis) of or assisting in the diagnosis of the possibility of developing an immune-mediated inflammatory disease.

In some embodiments, the above method is a method for predicting the possibility of developing the immune-mediated inflammatory disease characterized by an increase in expression of MMP12, in a subject, and the above method further includes comparing the level of MMP12 in the biological sample obtained from the subject with a reference value. If the level of MMP12 in the biological sample obtained from the subject is equal to or lower than the reference value, it indicates that there is a low possibility that the subject will develop the immune-mediated inflammatory disease characterized by an increase in expression of MMP12.

The above reference value is, for example, an average or median value calculated from measurement of the levels of MMP12 in a plurality of healthy subjects, or a value lower than these values.

If it is determined based on such a determination criterion that there is a low possibility that the subject will develop the immune-mediated inflammatory disease characterized by an increase in expression of MMP12, this determination result can be taken into account in determining a future treatment plan for the subject.

In some embodiments, the above method is a method for predicting the prognosis of a subject who has developed the immune-mediated inflammatory disease characterized by an increase in expression of MMP12, and the subject is a subject who has received treatment for the immune-mediated inflammatory disease characterized by an increase in expression of MMP12. The above method further includes comparing the level of MMP12 in the biological sample obtained from the subject with a reference value, and if the level of MMP12 in the biological sample obtained from the subject is higher than the reference value, it indicates that there is a high possibility of relapse of the immune-mediated inflammatory disease characterized by an increase in expression of MMP12, in the subject.

The subject who has received treatment for the immune-mediated inflammatory disease characterized by an increase in expression of MMP12 also includes a patient who has discontinued treatment for the immune-mediated inflammatory disease characterized by an increase in expression of MMP12 after receiving the treatment.

Examples of the treatment include administration of a drug useful for treating the immune-mediated inflammatory disease characterized by an increase in expression of MMP12, radiation therapy, exercise therapy, diet therapy, administration of supplements and the like.

The above reference value is, for example, the level of MMP12 in a sample obtained from the same subject before receiving treatment for treating the immune-mediated inflammatory disease characterized by an increase in expression of MMP12, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

If it is determined based on such a determination criterion that there is a high possibility that the subject will relapse into the immune-mediated inflammatory disease characterized by an increase in expression of MMP12, this determination result can be taken into account in determining a future treatment plan for the subject such as resuming administration of the therapeutic drug. The treatment on the subject can be more appropriately performed. An increase in the level of MMP12 precedes the onset of symptoms related to relapse of the immune-mediated inflammatory disease, and thus the method of the present embodiment is useful as a means for diagnosis (especially early diagnosis) of or assisting in the diagnosis of the possibility of relapse of an immune-mediated inflammatory disease.

The inventors found that MMP12 is positively correlated with the PET vascular activity score (PETVAS) which is used in PET-CT examinations. The method of the present embodiment can be a better evaluation method than PETVAS and is useful for stratifying the risk of relapse in the subject.

In some embodiments, the above method is a method for predicting the prognosis of a subject who has developed the immune-mediated inflammatory disease characterized by an increase in expression of MMP12, and the subject is a subject who has received treatment for the immune-mediated inflammatory disease characterized by an increase in expression of MMP12. The above method further includes comparing the level of MMP12 in the biological sample obtained from the subject with a reference value, and if the level of MMP12 in the biological sample obtained from the subject is equal to or lower than the reference value, it indicates that there is a low possibility of relapse of the immune-mediated inflammatory disease characterized by an increase in expression of MMP12, in the subject.

The subject who has received treatment for the immune-mediated inflammatory disease characterized by an increase in expression of MMP12 also includes a patient who has discontinued treatment for the immune-mediated inflammatory disease characterized by an increase in expression of MMP12 after receiving the treatment.

Examples of the treatment include administration of a drug useful for treating the immune-mediated inflammatory disease characterized by an increase in expression of MMP12, radiation therapy, exercise therapy, diet therapy, administration of supplements and the like.

The above reference value is, for example, the level of MMP12 in a sample obtained from the same subject before receiving treatment for treating the immune-mediated inflammatory disease characterized by an increase in expression of MMP12, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

The "cutoff value" can be obtained by various statistical analysis methods known to those skilled in the art. Examples of the "cutoff value" include the average or median value of the levels of MMP12 in a plurality of patients with the immune-mediated inflammatory disease characterized by an increase in expression of MMP12, a value at which a P-value is minimized or a value at which a P-value is less than a certain level (e.g., a value at which the P-value is less than 0.1, a value at which the P-value is less than 0.05) in a log-rank test for the effect of the above treatment that separates a group of healthy subjects and a group of patients, a value obtained based on ROC (Receive Operating Characteristic) analysis such that the sum of sensitivity and specificity is maximized based on the relationship between the expression amount of MMP12 in patients who have received the treatment and the effect of the treatment, and a value at which a P-value is minimized or a value at which a P-value is less than a certain level (e.g., a value at which the P-value is less than 0.1, a value at which the P-value is less than 0.05) in a chi-square test for the effect of the treatment in a group of healthy subjects and a group of patients.

If it is determined based on such a determination criterion that there is a low possibility that the subject will relapse into the immune-mediated inflammatory disease characterized by an increase in expression of MMP12, this determination result can be taken into account in determining a future treatment plan for the subject such as refraining from further treatment, which is advantageous from the viewpoint of the burden on the subject and economy.

In some embodiments, the above method is a method for predicting the post-treatment condition of a subject who has developed the immune-mediated inflammatory disease characterized by an increase in expression of MMP12, and the subject is a subject who has received treatment for the immune-mediated inflammatory disease characterized by an increase in expression of MMP12. The above method further includes comparing the level of MMP12 in the biological sample obtained from the subject with a reference value, and if the level of MMP12 in the biological sample obtained from the subject is lower than the reference value, it indicates that there is a high possibility that the immune-mediated inflammatory disease characterized by an increase in expression of MMP12, in the subject, has remitted.

Examples of the treatment include administration of a drug useful for treating the immune-mediated inflammatory disease characterized by an increase in expression of MMP12, radiation therapy, exercise therapy, diet therapy, administration of supplements and the like.

Examples of the drug useful for treating the immune-mediated inflammatory disease characterized by an increase in expression of MMP12 include, but are not limited to, steroids (prednisolone (registered trademark)), cyclophosphamide (Endoxan (registered trademark)), methotrexate (Rheumatrex (registered trademark)), rituximab (Rituxan (registered trademark)), avacopan (TAVNEOS (registered trademark)), tocilizumab (Actemra (registered trademark)) and the like.

The above reference value is, for example, the level of MMP12 in a sample obtained from the same subject before receiving treatment for treating the immune-mediated inflammatory disease characterized by an increase in expression of MMP12, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

If it is determined based on such a determination criterion that there is a high possibility that the immune-mediated inflammatory disease characterized by an increase in expression of MMP12, in the subject, has remitted, the effectiveness of the treatment received by the subject can be evaluated, and this determination result can be taken into account in determining a future treatment plan for the subject such as refraining from administering the therapeutic drug, which is advantageous from the viewpoint of the burden on the subject and economy.

In some embodiments, the above method is a method for evaluating the timing of administration of a therapeutic drug for the immune-mediated inflammatory disease characterized by an increase in expression of MMP12, to a subject with the immune-mediated inflammatory disease characterized by an increase in expression of MMP12. The above method further includes comparing the level of MMP12 in the biological sample obtained from the subject with a reference value, and a fact that the level of MMP12 in the biological sample obtained from the subject is lower than the reference value is an indicator for discontinuing the administration of the drug to the subject.

The above reference value is, for example, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

If it is determined based on such a determination criterion that the level of MMP12 in the biological sample obtained from the subject is lower than the reference value, it is determined that it is appropriate to discontinue the administration of the drug to the subject, and this determination result can be taken into account in determining a future treatment plan for the subject such as refraining from administering the therapeutic drug, which is advantageous from the viewpoint of the burden on the subject and economy.

In some embodiments, the above method is a method for determining the therapeutic effect of treatment in a subject with the immune-mediated inflammatory disease characterized by an increase in expression of MMP12, and the subject is a subject who has received treatment for the immune-mediated inflammatory disease characterized by an increase in expression of MMP12. The above method further includes comparing the level of MMP12 in the biological sample obtained from the subject with a reference value, and if the level of MMP12 in the biological sample obtained from the subject is lower than the reference value, it indicates that the therapeutic effect of the treatment on the immune-mediated inflammatory disease characterized by an increase in expression of MMP12 in the subject is high.

Examples of the treatment include administration of a drug useful for treating the immune-mediated inflammatory disease characterized by an increase in expression of MMP12, radiation therapy, exercise therapy, diet therapy, administration of supplements and the like.

The reference value is, for example, the level of MMP12 in a sample obtained from the same subject before receiving treatment for treating the immune-mediated inflammatory disease characterized by an increase in expression of MMP12, a cutoff value that separates a group of healthy subjects and a group of patients diagnosed as having the immune-mediated inflammatory disease characterized by an increase in expression of MMP12 based on a doctor's diagnosis (e.g., within the range of 50 pg/ml to 200 pg/ml, preferably 100 pg/ml), or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12 (e.g., within the range of 250 pg/ml to 1000 pg/ml, preferably 500 pg/ml).

If it is determined based on such a determination criterion that the therapeutic effect of the treatment in the subject on the immune-mediated inflammatory disease characterized by an increase in expression of MMP12 is high, the effectiveness of the treatment received by the subject can be evaluated, and this determination result can be taken into account in determining a future treatment plan for the subject such as refraining from administering the therapeutic drug, which is advantageous from the viewpoint of the burden on the subject and economy.

According to a second embodiment of the present invention, a biomarker for diagnosing or detecting an immune-mediated inflammatory disease characterized by an increase in expression of MMP12, including MMP12, is provided.

In some embodiments, MMP12 in a biological sample obtained from a subject is used as a biomarker for diagnosing or detecting an immune-mediated inflammatory disease characterized by an increase in expression of MMP12.

According to the third embodiment of the present invention, a diagnostic drug for an immune-mediated inflammatory disease characterized by an increase in expression of MMP12, containing a substance that specifically interacts with MMP12, is provided. The term "specifically interacting" means an interaction that is different from a non-specific interaction to a measurable extent, and means interacting with each other.

Examples of the substance that specifically interacts with MMP12 include the substances that bind to MMP12 and the substances that are cleaved by MMP12 due to the protease activity of MMP12, as described in the first embodiment, such as peptides that have sites that can be cleaved by MMP12.

In some embodiments, the diagnostic drug includes a substance that binds to MMP12, and the substance that binds to MMP12 can be selected from the group consisting of an antibody to MMP12, an antigen-binding fragment of an antibody to MMP12, and an aptamer to MMP12.

In some embodiments, the diagnostic drug utilizes the protease activity of MMP12 and includes a conjugate of a peptide having a site that can be cleaved by MMP12 as a substrate site for MMP12 and a label substance. The peptide having a site that can be cleaved by MMP12 and the label substance may be directly bound or bound via a linker. The peptide having a site that can be cleaved by MMP12 and the label substance can be bound via or without a linker by any known method. The linker can also be referred to interchangeably as "spacer". Examples of the linker include, but are not limited to, saturated hydrocarbon groups having 5 to 12 carbon atoms and 1 to 5 amino acids that may have substituents. The linker can be bound to one or both of the N-terminal and C-terminal of the peptide. Linkers that separate the peptide and the label substance that are recognized by the protein to be detected are known, and such linkers can be easily selected by those skilled in the art. The diagnostic drug may be an in vitro diagnostic drug or an in vivo diagnostic drug.

The length of the peptide having a site that can be cleaved by MMP12 is not particularly limited, and in terms of MMP12 detection sensitivity, it is preferred that the peptide is composed of a sequence of 8 to 25 amino acids, it is more preferred that the peptide is composed of a sequence of 10 to 20 amino acids, and it is further preferred that the peptide is composed of a sequence of 12 to 18 amino acids.

The peptide having a site that can be cleaved by MMP12 can be determined based on the peptide sequences of proteins known to be selective substrates for MMP12. Examples of the proteins known to be selective substrates for MMP12 include human CCL-14 (UniPlot Q16627), mouse CXCL1 (UniPlotA2RTH0), mouse CXCL2 (UniPlot P12850), mouse CXCL3 (UniPlot P10889), mouse CXCL5 (UniPlot P50228), human CXCL1 (UniPlot P09341), human CXCL2 (UniPlot P19875), human CXCL3 (UniPlot P19876), human CXCL5 (UniPlot P42830), human CXCL6 (P80162), human CXCL7 (UniPlot P02775), human CXCL8 (UniPlot P10145), human INF-α-2 (UniPlot P01563), human INF-α-6 (UniPlotA0A0A0MQU8), human INF-α-8 (UniPlot P32881), human INF-α-10 (UniPlot P01566), human IFN-γ (UniPlot P01579), mouse IFN-γ (UniPlot P01580) and the like. See, for example, J Biol Chem 2012, 287, 5848-60, Blood 2008, 112, 3455-64, Nat Med. 2014, 20, 493-502, and Nat Commun. 2018, 9, 2416.

In some embodiments, the peptide having a site that can be cleaved by MMP12 includes a consecutive sequence of 8 to 25 amino acids, preferably a consecutive sequence of 10 to 20 amino acids, and more preferably a consecutive sequence of 12 to 18 amino acids, including an amino acid sequence having a site that can be cleaved by MMP12, in a protein selected from among human CCL-14 (hCCL-14), mouse CXCL1 (mCXCL1), mouse CXCL2 (mCXCL2), mouse CXCL3 (mCXCL3), mouse CXCL5 (mCXCL5), human CXCL1 (hCXCL1), human CXCL2 (hCXCL2), human CXCL3 (hCXCL3), human CXCL5 (hCXCL5), human CXCL6 (hCXCL6), human CXCL7 (hCXCL7), human CXCL8 (hCXCL8), human INF-α-2 (hlNF-α-2), human INF-α-6 (hINF-α-6), human INF-α-8 (hINF-α-8), human INF-α-10 (hINF-α-10), human IFN-γ(hIFN-γ), and mouse IFN-γ(mIFN-γ). However, in the natural sequence in the peptide, an amino acid that is cysteine (Cys) may be substituted with another amino acid such as serine (Ser).

The sites in the peptide that are specifically recognized by MMP12 and can be cleaved by MMP12 are known, and examples of the sites include, but are not limited to, ELRCXC (cleavage between E and LRCXC, X is preferably Q or V), PLGLAG (cleavage between PLG and LAG, see J.Am.Chem.Soc., 2012, 134, 13730-1373), PLGLR (cleavage between LG and LR, see Chemistry and Biology, 2015, 22, 1122-1133), FGALT (cleavage between FGA and LT, ibid.), VYDLK (cleavage between VYD and LK, ibid.), PWAWR (cleavage between PWA and WR, ibid.) and the like.

The amino acid sequence of the peptide having a site that can be cleaved by MMP12 is preferably the same as or similar to the natural amino acid sequence in terms of specificity for MMP12.

Examples of the peptide having a site that can be cleaved by MMP12 and having an amino acid sequence that is the same as or similar to the natural amino acid sequence include a partial peptide or a variant thereof that includes the ELRCXC sequence of CXC (α-chemokine), which is a subfamily of chemokine proteins.

In some embodiments, the peptide having a site that can be cleaved by MMP12 is a peptide composed of a sequence of 8 to 25 amino acids having an amino acid sequence of ELRCXC (X is Q or V). The sequences upstream and downstream of ELRCXC can be designed using ordinary techniques by a person skilled in the art based on the amino acid sequence of the CXC protein under conditions where the peptide has the ability to be cleaved by MMP12.

In some embodiments, the peptide having a site that can be cleaved by MMP12 is either the following (i) or (ii): (i) a peptide having any of partial sequences of the amino acid sequences of human CXCL1 (UniPlot P09341), human CXCL2 (UniPlot P19875), human CXCL3 (UniPlot P19876), human CXCL5 (UniPlot P42830), human CXCL8 (UniPlot P10145), mouse CXCL1 (UniPlotA2RTH0), mouse CXCL2 (UniPlot P12850), mouse CXCL3 (UniPlot P10889), and mouse CXCL5 (UniPlot P50228), having an amino acid sequence of ELRCXC (X is Q or V), having a sequence of 1 to 5 amino acids upstream thereof, having a sequence of one or more amino acids downstream thereof, and composed of a sequence of 8 to 25 amino acids as a total length; and (ii) a peptide in which, in the peptide of (i) above, further, one or more of the amino acids upstream of the amino acid sequence of ELRCXC (X is Q or V) are substituted, one or more of the amino acids downstream of the amino acid sequence of ELRCXC (X is Q or V) are substituted, or one or more of the amino acids upstream of the amino acid sequence of ELRCXC (X is Q or V) are substituted, and one or more of the amino acids downstream of the amino acid sequence of ELRCXC (X is Q or V) are substituted.

In some embodiments, the peptide having a site that can be cleaved by MMP12 is any of the following (i) to (vi): (i) a peptide having any of the amino acid sequences of SEQ ID NOs: 1 to 9; (ii) a peptide composed only of any of the amino acid sequences of SEQ ID NOs: 1 to 9; (iii) a peptide composed of a partial sequence of a peptide having any of the amino acid sequences of SEQ ID NOs: 1 to 9 having a part of ELRCXC (X is Q or V); and (iv) a peptide in which, in the peptides of (i) to (iii) above, further, one or more of the first to fifth amino acids of any of the amino acid sequences of SEQ ID NOs: 1 to 9 are substituted, one or more of the 12th and 13th amino acids of any of the amino acid sequences of SEQ ID NOs: 1 to 9 are substituted, or both one or more of the first to fifth amino acids of any of the amino acid sequences of SEQ ID NOs: 1 to 9 and one or more of the 12th and 13th amino acids of any of the amino acid sequences of SEQ ID NOs: 1 to 9 are substituted.
Amino acid sequence of a peptide derived from human CXCL1 ASVATELRCQCLQ (SEQ ID NO: 1)
Amino acid sequence of a peptide derived from human CXCL2 APLATELRCQCLQ (SEQ ID NO: 2)
Amino acid sequence of a peptide derived from human CXCL3 ASVVTELRCQCLQ (SEQ ID NO: 3)
Amino acid sequence of a peptide derived from human CXCL5 AAVLRELRCVCLQ (SEQ ID NO: 4)
Amino acid sequence of a peptide derived from human CXCL8 PRSAKELRCQCIK (SEQ ID NO: 5)
Amino acid sequence of a peptide derived from mouse CXCL1 APIANELRCQCLQ (SEQ ID NO: 6)
Amino acid sequence of a peptide derived from mouse CXCL2 AWASELRCQCLK (SEQ ID NO: 7)
Amino acid sequence of a peptide derived from mouse CXCL3 AVVASELRCQCLN (SEQ ID NO: 8)
Amino acid sequence of a peptide derived from mouse CXCL5 VIAATELRCVCLT (SEQ ID NO: 9)

In some embodiments, the peptide having a site that can be cleaved by MMP12 is any of the following (i) to (v): (i) a peptide having any of the amino acid sequences of SEQ ID NOs: 10 to 32; (ii) a peptide composed only of any of the amino acid sequences of SEQ ID NOs: 10 to 32; (iii) a peptide composed of a partial sequence of a peptide having any of the amino acid sequences of SEQ ID NOs: 10 to 32 having a site that is cleaved by MMP12, and composed of a sequence of 8 to 25 amino acids as a total length; (iv) a peptide in which, in any of the peptides of (i) to (iii) above, one or more of amino acids other than the amino acids having a site that is cleaved by MMP12 are substituted; and (v) a peptide in which, in any of the peptides of (i) to (iii) above, all the cysteines in the natural sequence are substituted with other amino acids such as serine.

Any of the peptides of (i) to (v) is a peptide composed of a sequence of 8 to 25 amino acids, preferably a peptide composed of a sequence of 10 to 20 amino acids, and more preferably a peptide composed of a sequence of 12 to 18 amino acids.

Amino acid sequence at positions 1 to 12 of hCCL-14 TKTESSSRGPYH (Sequence No. 10) T-E at positions 3-4 and S-S at positions 6-7 are cleavable sites.

Amino acid sequence at positions 61-74 of hCCL-14 DKWVQDYIKDMKEN (SEQ ID NO: 11) M-K at positions 11-12 and K-E at positions 12-13 are cleavable sites.

Amino acid sequence at positions 154-165 of hIFN-α-10 STNLQKRLRRKD (SEQ ID NO: 12) L-Q at positions 4-5 and L-R at positions 8-9 are cleavable sites.

Amino acid sequence at positions 150-161 of hIFN-α-10 SLSFSTNLQKRL (SEQ ID NO: 13) L-Q at positions 8-9 is a cleavable site.

Amino acid sequence at positions 154-165 of hIFN-α-6 SRNLQERLRRKE (SEQ ID NO: 14) L-Q at positions 4-5 and L-R at positions 8-9 are cleavable sites.

Amino acid sequence at positions 150-161 of hIFN-α-6 SFSSSRNLQERL (SEQ ID NO: 15) L-Q at positions 8-9 is a cleavable site.

Amino acid sequence at positions 154-165 of hIFN-α-2 STNLQESLRRKE (SEQ ID NO: 16) L-Q at positions 4-5 and L-R at positions 8-9 are cleavable sites.

Amino acid sequence at positions 150-161 of hIFN-α-2 SFSLSTNLQESL (SEQ ID NO: 17) L-Q at positions 8-9 is a cleavable site

Amino acid sequence at positions 154-165 of hIFN-α-8 SINLQKRLKSKE (SEQ ID NO: 18) L-Q at positions 4-5 and L-K at positions 8-9 are cleavable sites.

Amino acid sequence at positions 150-161 of hIFN-α-8 SFSLSINLQKRL (SEQ ID NO: 19) L-Q at positions 8-9 is a cleavable site.

Amino acid sequence at positions 126-139 of hIFN-γ LNVQRKAIHELIQV (SEQ ID NO: 20) E-L at positions 10-11 is a cleavable site.

Amino acid sequence at positions 130-143 of hIFN-γ RKAIHELIQVMAEL (SEQ ID NO: 21) E-L at positions 6-7 is a cleavable site.

Amino acid sequence at positions 151-165 of hIFN-γ KRKRSQMLFRGRRAS (SEQ ID NO: 22) M-L at positions 7-8 is a cleavable site.

Amino acid sequence at positions 125-137 of mIFN-γ QVQRQAFNEKIRV (SEQ ID NO: 23) E-L at positions 9-10 is a cleavable site.

Amino acid sequence at positions 128-141 of mIFN-γ RQAFNEKIRVVHQL (SEQ ID NO: 24) E-L at positions 6-7 is a cleavable site.

Amino acid sequence at positions 143-154 of mIFN-γ PESSLRKRKRSR (SEQ ID NO: 25) R-K at positions 8-9 is a cleavable site.

Amino acid sequence at positions 1-14 of mCXCL1 APIANELRSQSLQT (SEQ ID NO: 26) P-I at positions 2-3 and E-L at positions 6-7 are cleavable sites.

Amino acid sequence at positions 78-90 of mCXCL5 AKRNALAVERTAS (SEQ ID NO: 27) A-V at positions 7-8 is a cleavable site.

Amino acid sequence at positions 76-89 of hCXCL7 APRIKKIVQKKLAG (SEQ ID NO: 28) K-I at positions 6-7 is a cleavable site.

Amino acid sequence at positions 1-14 of hCXCL3 ASVVTELRSQSLQT (SEQ ID NO: 29) E-L at positions 6-7 is a cleavable site.

Amino acid sequence at positions 3-14 of hCXCL8 LPRSAKELRSQS (SEQ ID NO: 30) L-R at positions 8-9 is a cleavable site.

Amino acid sequence at positions 3-15 of hCXCL5 PAAAVLRELRSVS (SEQ ID NO: 31) E-L at positions 8-9 is a cleavable site.

Amino acid sequence at positions 9-22 of hCXCL6 ELRSTSLRVTLRVN (SEQ ID NO: 32) S-L at positions 6-7 is a cleavable site.

In a specific preferred embodiment, the peptide having a site that can be cleaved by MMP12 is any of the following (i) to (v): (i) a peptide having any of the amino acid sequences of SEQ ID NOs: 11, 13, 14, 15, 16, 20, 24, 25, 26, 27, 29, and 31; (ii) a peptide composed only of any of the amino acid sequences of SEQ ID NOs: 11, 13, 14, 15, 16, 20, 24, 25, 26, 27, 29, and 31; (iii) a peptide composed of a partial sequence of a peptide having any of the amino acid sequences of SEQ ID NOs: 11, 13, 14, 15, 16, 20, 24, 25, 26, 27, 29, and 31 having a site that is cleaved by MMP12, and composed of a sequence of 8 to 25 amino acids as a total length; (iv) a peptide in which, in any of the peptides of (i) to (iii) above, one or more of amino acids other than the amino acids having a site that is cleaved by MMP12 are substituted; and (v) a peptide in which, in any of the peptides of (i) to (iii) above, all the cysteines in the natural sequence are substituted with other amino acids such as serine.

Examples of the label substance include radioactive nuclides, enzymes, fluorescent dyes, contrast agents (e.g., paramagnetic ions) and the like.

Examples of radioactive nuclides include [³H], [¹¹C], [¹⁴C], [¹⁸F], [³⁴Cl], [³⁸Cl], [⁷⁵Br], [⁷⁶Br], [⁷⁷Br], [⁸⁰Br], [⁸²Br], [¹²¹I], [¹²³I], [¹²⁴I], [¹²⁵I], [¹²⁷I], [¹³¹I], [⁶⁴Cu], [⁹⁹Y], [⁶⁷Ga], [⁵¹Cr], [¹⁹²Ir], [⁹⁹Mo], [¹⁵³Sm], [²⁰¹Tl] and the like.

Examples of fluorescent dyes include, but are not limited to, phenyl and derivatives thereof, naphthalene and derivatives thereof such as 5-dimethylaminonaphthalene-1-sulfonic acid and hydroxynaphthalenes, anthracene and derivatives thereof such as 9,10-diphenylnaphthalene and 9-methylanthracene, pyrene and derivatives thereof such as N-(1-pyrene)iodoacetamide and hydroxypyrenes, biphenyl and derivatives thereof, acridine and derivatives thereof such as hydroxyacridines and 9-methylacridine, coumarin and derivatives thereof such as 7-dialkylamino-4-methylcoumarin and 4-bromomethyl-7-methoxycoumarin, xanthene and derivatives thereof, phthalocyanine and derivatives thereof, stilbene and derivatives thereof such as 6,6'-dibromostilbene and hydroxystilbenes, furan and derivatives thereof, oxazole and derivatives thereof, oxadiazole and derivatives thereof, nitrobenzoxadiazole and derivatives thereof such as hydroxynitrobenzoxadiazoles, benzothiazole and derivatives thereof, fluorescein and derivatives thereof such as 5-iodoacetamidofluorescein and fluorescein-5-maleimide, rhodamine and derivatives thereof such as tetramethylrhodamine, tetraethyl rhodamine, and carboxy tetramethylrhodamine, BODIPY (registered trademark) and derivatives thereof, eosin and derivatives thereof, erythrosine and derivatives thereof such as hydroxyerythrosines and 5-iodoacetamidoerythrosine, resorufin and derivatives thereof such as hydroxyresorufin, quinoline and derivatives thereof such as 6-hydroxyquinoline and 6-aminoquinoline, carbazole and derivatives thereof such as N-methylcarbazole, cyanine and derivatives thereof such as hydroxycyanine, carbocyanine and derivatives such as phenylcarbocyanine, pyridinium salts and derivatives thereof such as 4-(4-dialkyldiamidostyryl)-N-methylpyridinium iodide, fluorescent complexes of lanthanide and derivatives thereof, green fluorescent protein (GFP) and derivatives thereof, benzoindole and derivatives thereof (such as indocyanine green) and the like. The derivatives are each preferably a compound in which the carbon skeleton of the compound before derivatization is maintained. The fluorescent dyes and derivatives thereof listed above can be synthesized, but commercially available products can be used.

A fluorescent dye having a maximum excitation wavelength in the range of 400 nm to 1200 nm is suitable for optical imaging, and a fluorescent dye having a maximum excitation wavelength (which may also be an absorption maximum wavelength) in the near-infrared region of 650 nm to 900 nm is preferable from the viewpoint of enabling fluorescent imaging of deep tissue portions in the subject. Fluorescent dyes such as fluorescein and derivatives thereof, rhodamine and derivatives thereof, BODIPY (registered trademark) and derivatives thereof, and cyanine and derivatives thereof can be advantageously selected from molecules having a fluorescent emission wavelength in the range of 400 to 900 nanometers, particularly 500 to 900 nanometers, and further particularly 600 to 900 nanometers.

Specific fluorescent dyes are the following compounds.

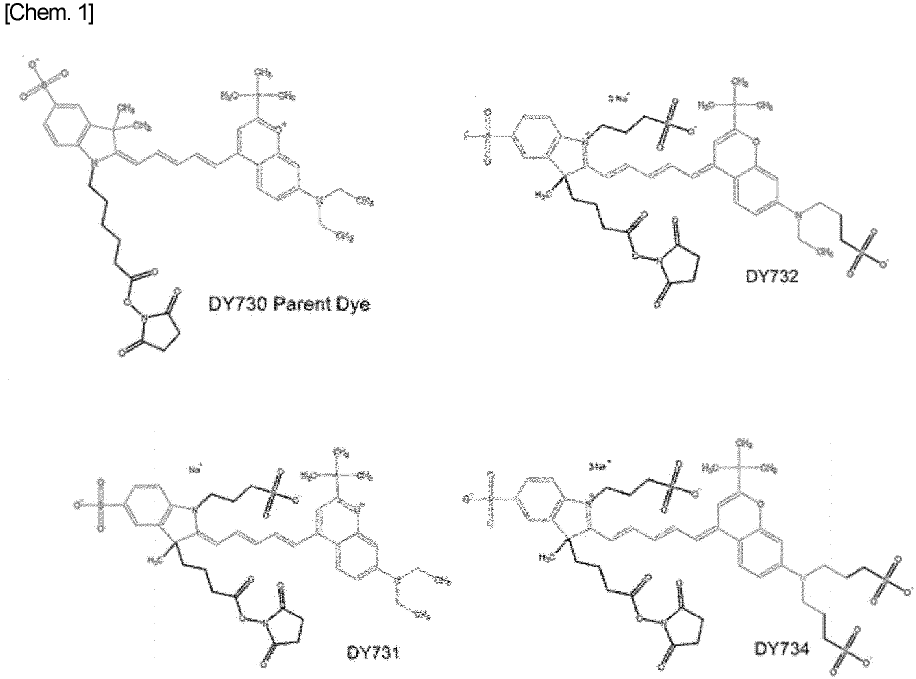

In some embodiments, the diagnostic drug is a fluorescent probe for detecting MMP12, including a peptide having a site that can be cleaved by MMP12 and a fluorophore bound to the peptide. The peptide having a site that can be cleaved by MMP12 and the fluorophore may be directly bound or bound via a linker. The peptide having a site that can be cleaved by MMP12 can be synthesized by a known method. The fluorophore can be formed by binding any of the fluorescent dyes listed above to the middle or end of the amino acid sequence of the peptide.

In some embodiments, the diagnostic drug is a fluorescent probe for detecting MMP12, composed of a conjugate including a peptide having a site that can be cleaved by MMP12, a fluorophore bound to one end of the peptide, and a quencher bound to the other end of the peptide. The peptide having a site that can be cleaved by MMP12 and the quencher may be directly bound or bound via a linker. Examples of the linker include, but are not limited to, saturated hydrocarbon groups having 5 to 12 carbon atoms and 3 to 5 amino acids that may have substituents. The quencher can be formed by binding a quenching compound to the middle or end of the amino acid sequence of the peptide.

As the combination of the fluorophore and the quencher, a combination known in the art, which utilizes a fluorescence control principle by fluorescence resonance energy transfer (FRET), can be used, in which when both the fluorophore and the quencher exist in a fluorescent probe molecule, fluorescence is quenched by the quencher suppressing the fluorescence of the fluorophore, but the fluorescence is increased by cleavage of the amino acid sequence of the peptide.

Examples of the fluorophore include a fluorophore formed by binding the fluorescent dye as the above label substance to the peptide, and preferably include compounds, including fluorescein and derivatives thereof, cyanine and derivatives thereof, rhodamine and derivatives thereof, BODIPY (registered trademark) and derivatives thereof, coumarin and derivatives thereof, anthracene and derivatives thereof, aminobenzyl and the like.

The quencher is a compound or part thereof that causes the fluorescent dye to lose its fluorescence through energy transfer, stacking and the like., when being spatially close to the fluorophore.

Examples of the quenching compound include compounds in which phenyl groups are bound to the nitrogen atoms at positions 3 and 6 of a xanthene ring (e.g., QSY series compounds from ThermoFisher Scientific), compounds in which aromatic rings are bound to the nitrogen atoms at positions 3 and 6 of the xanthene ring of a rhodamine compound (SiR dye) in which the oxygen atom of rhodamine or a derivative thereof is substituted with a silicon atom, to make the compound non-fluorescent with a fluorescence quantum yield of 0.001 or less (e.g., SinQ series compounds invented by Dr. Kenjiro Hanaoka, who is one of the inventors, see J. Am. Chem. Soc., 137, 4759-4765 (2015)), and compounds containing dinitrophenol (e.g., Black Hole Quencher (BHQ) series compounds from Sigma-Aldrich).

A specific quenching compound is the following compound.

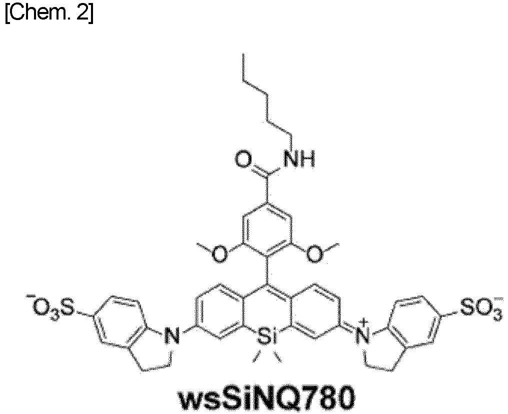

The binding of each of the fluorescent dye and the quenching compound to the peptide having a site that can be cleaved by MMP12 can be carried out by a known method, such as by reaction of each of the functional groups (for example, carboxyl group, amino group) of the fluorescent dye and the quenching compound with the functional groups of the terminal amino acids of the peptide, or by formation of a covalent bond via a linker. The efficiency of cleavage of the peptide by MMP can be analyzed by high performance liquid chromatography. An amino acid sequence having high cleavage activity and selectivity is determined and completed as a probe.

In some embodiments, the immune-mediated inflammatory disease characterized by an increase in expression of MMP12 is selected from the group consisting of vasculitis syndrome, granulomatous vasculitis, IgG4, inflammatory bowel disease, sarcoidosis, tuberculous mycobacterial infection, and nontuberculous mycobacterial infection.

According to a fourth aspect of the present invention, a diagnostic or detection kit for diagnosing or detecting an immune-mediated inflammatory disease characterized by an increase in expression of MMP12, including a substance that specifically interacts with MMP12, is provided.

As the substance that specifically interacts with MMP12, the substance that specifically interacts with MMP12 as described for the diagnostic drug of the third embodiment can be used. The label substance described in the first or third embodiment may be bound to the substance that specifically interacts with MMP12. Alternatively, the diagnostic drug of the third embodiment may be used as the substance that specifically interacts with MMP12.

In addition to the substance that specifically interacts with MMP12, the diagnostic or detection kit may include another substance that is necessary for the reaction to detect the level of MMP12 and does not have an adverse effect on the reaction when stored in a coexistent state, for example, various reagents such as coloring reagents, labeled secondary antibodies, and blocking agents. The diagnostic or detection kit may further include a buffer, a washing solution, an instruction manual or the like.

The substance that specifically interacts with MMP12 may be provided in a state of being immobilized on a suitable solid support. Examples of the solid support include various carriers made of insoluble polysaccharides (e.g., agarose, dextran, or cellulose), synthetic resins, glass, and metals, which are used for ordinary antigen-antibody reactions, such as microplates, tubes, membranes, columns, beads, and sensor chips. For the immobilization, physical adsorption may be used, or chemical bonding, which is usually used to insolubilize and immobilize proteins, may be used.

The diagnostic or detection kit of the present invention can be applied to clinical tests such as diagnosis, detection, or monitoring of immune-mediated inflammatory diseases characterized by an increase in expression of MMP12, and can be used for drug discovery research.

In some embodiments, the substance that specifically interacts with MMP12 is a substance that binds to MMP12, selected from the group consisting of an antibody to MMP12, an antigen-binding fragment of an antibody to MMP12, and an aptamer to MMP12. The diagnostic or detection kit including such a substance that binds to MMP12 can be used as an in vitro diagnostic or detection kit.

In some embodiments, the substance that specifically interacts with MMP12 is a substance that is cleaved by MMP12 due to the protease activity of the MMP12, for example, a peptide having a site that can be cleaved by MMP12. The label substance described in the third embodiment may be bound to the substance that is cleaved by MMP12, or a fluorophore and a quencher may be bound to the substance that is cleaved by MMP12. The diagnostic or detection kit including such a substance that is cleaved by MMP12 due to the protease activity of the MMP12 can be used as an in vivo diagnostic or detection kit.

According to a fifth embodiment of the present invention, provided is a method for screening for a substance effective for treatment for an immune-mediated inflammatory disease characterized by an increase in expression of MMP12, the method including: measuring the level of MMP12 in a biological sample obtained from a subject after administration of a test substance; and if the level of MMP12 is decreased due to the administration of the test substance, selecting the test substance as a candidate substance effective for treatment for the immune-mediated inflammatory disease characterized by an increase in expression of MMP12.

The biological sample in which MMP12 has been expressed may be a body fluid, tissue, or cell. The subject is preferably a human with the immune-mediated inflammatory disease characterized by an increase in expression of MMP12 or a non-human mammal model of the immune-mediated inflammatory disease characterized by an increase in expression of MMP12. The level of MMP12 can be measured using the same method as described in the first embodiment of the present invention.

If the level of MMP12 in the biological sample obtained from the subject to which the test substance has been administered is compared with a control value and is lower than the control value, it is determined that the MMP12 in the biological sample has been decreased by the test substance, and the test substance can be selected as a candidate substance effective for treatment for the immune-mediated inflammatory disease characterized by an increase in expression of MMP12.

The control value is the level of MMP12 in a biological sample obtained from the same subject before administration of the test substance, an average or median value calculated from measurement of the levels of MMP12 in a plurality of healthy subjects, a cutoff value that separates a group of healthy subjects and a group of patients diagnosed as having the immune-mediated inflammatory disease characterized by an increase in expression of MMP12, or a certain value that is higher than an average or median value calculated from measurement of the levels of MMP12 in a plurality of healthy subjects and is lower than an average or median value calculated from measurement of the levels of MMP12 in a plurality of patients with the immune-mediated inflammatory disease characterized by an increase in expression of MMP12.

According to a sixth embodiment of the present invention, a therapeutic agent for an immune-mediated inflammatory disease characterized by an increase in expression of MMP12, including an MMP12 inhibitory substance, is provided.

MMP12 has an action of degrading extracellular matrices such as collagen, elastin, fibronectin, fibrin, fibrinogen, and proteoglycan and causes rupture of connective tissues such as elastic plates of vessel walls and glomeruli.

The MMP12 inhibitory substance refers to a substance that binds to MMP12 and inhibits the substrate degradation action of MMP12 and subsequent tissue damage, and a substance that is the same as described for the "substance that binds to MMP12" in the method of the first embodiment can be used as the MMP12 inhibitory substance. The MMP12 inhibitory substance can be contained as an active ingredient of the therapeutic agent.

The MMP12 inhibitory substance may be a commercially available MMP12 inhibitor or may be an MMP12 inhibitor obtained or produced by a known method described in literature. Zinc is required for activation of MMP12, and in some embodiments, the MMP12 inhibitory substance is an MMP12 inhibitory substance that inhibits binding of zinc to the active center region of MMP12 or the catalytic action of MMP12 by zinc. Examples of the MMP12 inhibitor include, but are not limited to, selective MMP12 inhibitors such as MMP408 (CAS No. 1258003-93-8) and RXP470.1 (CAS No. 891198-31-5).

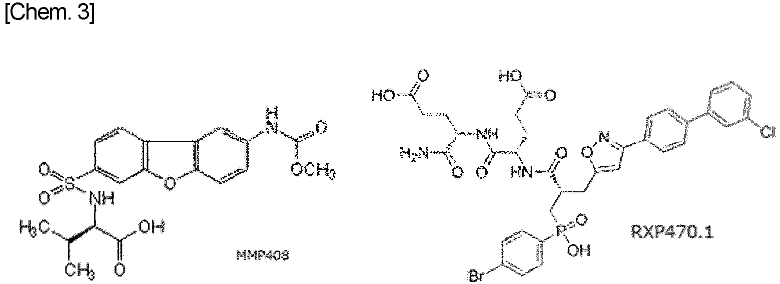

In some embodiments, the above MMP12 inhibitory substance can be selected from the group consisting of an antibody to MMP12 (anti-MMP12 antibody), an antigen-binding fragment of an anti-MMP12 antibody, and an aptamer to MMP12.

The administration route of the therapeutic agent for the immune-mediated inflammatory disease characterized by an increase in expression of MMP12, containing the MMP12 inhibitory substance, according to the present embodiment is not particularly limited, and the therapeutic agent may be an oral administration agent or a parenteral administration agent. For example, the therapeutic agent can be in the form of an oral administration agent such as a tablet, capsule, granule, powder, or syrup. In addition, the therapeutic agent can be in the form of a parenteral administration agent such as an injection, ophthalmic preparation, nasal drop, ointment, cream, lotion, gel, and spray. These pharmaceutical preparations can be manufactured by known methods.

For example, in the case of a pharmaceutical preparation for oral administration, the pharmaceutical preparation can be manufactured by formulating with an appropriate combination of: a solubilizer such as tragacanth gum, gum arabic, sucrose fatty acid esters, lecithin, olive oil, soybean oil, and PEG400; an excipient such as starch, mannitol, and lactose; a binder such as methylcellulose, sodium carboxymethylcellulose, and hydroxypropylcellulose; a disintegrant such as crystalline cellulose and calcium carboxymethylcellulose; a lubricant such as talc and magnesium stearate; and a flow improver such as light anhydrous silicic acid.

In addition, a typical example of a pharmaceutical preparation for parenteral administration is an injection. For example, a pharmaceutical preparation for injection can be prepared by dissolving or diluting an anti-MMP12 antibody or an aptamer to MMP12, which are MMP12 inhibitory substances, in a saline or buffer for intravenous injection. When the solubility of the MMP12 inhibitory substance is to be increased, it is possible to use known methods, such as changing the solvent, changing the salt contained in the solution, changing the ionic strength, or including the MMP12 inhibitory substance in a cyclodextrin, as appropriate. In addition to the MMP12 inhibitory substance, a pH regulator, a buffer, a stabilizer, an isotonizing agent, and a local anesthetic, can be added, and subcutaneous, intramuscular, and intravenous injections can be manufactured using ordinary methods. As an ophthalmic preparation, any of aqueous eye drops, non-aqueous eye drops, suspension eye drops, emulsion eye drops, and eye ointments, can be used. Such a pharmaceutical preparation can be prepared by blending a pharmaceutically acceptable carrier, particularly a carrier acceptable for ophthalmic preparations, for example, an isotonizing agent, a chelating agent, a stabilizer, a pH adjuster, a preservative, an antioxidant, a solubilizing agent, and a thickening agent., if necessary, in a composition suitable for the administration form, using a preparation method for a pharmaceutical preparation known to those skilled in the art. When preparing an ophthalmic preparation, the MMP12 inhibitory substance can be dissolved or suspended in an aqueous solvent such as sterile purified water or a saline, or a non-aqueous solvent such as vegetable oil such as cottonseed oil, soybean oil, sesame oil, or peanut oil, then adjusted to a predetermined osmotic pressure, and sterilized by filtration or the like. As an aqueous base for ophthalmic preparations, additives that are normally used, such as an isotonizing agent, a buffer, and a preservative, are blended as appropriate. Examples of the isotonizing agent include sodium chloride, potassium chloride, polyhydric alcohols, and saccharides., examples of the buffer include sodium borate, sodium citrate, monosodium phosphate, and disodium hydrogen phosphate., and examples of the preservative include benzethonium chloride, benzalkonium chloride, and chlorobutanol. In addition, a stabilizer such as glycerin or polysorbate 80, and a pH adjuster, can be blended if necessary. A nasal drop and a spray are prepared as liquid pharmaceutical preparations containing the MMP12 inhibitory substance. It is preferred that a nasal drop is placed in a container having a shape suitable for application to the nasal cavity. A spray is prepared in a state of being contained in a spray container containing a propellant together with a liquid pharmaceutical preparation. The propellant is a gas such as carbon dioxide. A spray may be used not only on the skin but also in the nasal cavity and oral cavity, and a spray container having a shape suitable for the method of use is selected as appropriate when preparing the spray.

The effective dose of the MMP12 inhibitory substance is changed as appropriate according to circumstances such as the condition and symptoms of the patient. The effective dosage can be determined usually within the range of 0.001 to 10 mg/kg/day and preferably within the range of 0.01 to 1 mg/kg/day as appropriate. However, the effective dosage can be adjusted according to the administration form, such as high dose for systemic administration and low dose for local administration. When administered orally, the daily dose of the MMP12 inhibitory substance can be administered once a day or in several doses. Conversely, the dose for several days can be administered at once, whereby the MMP12 inhibitory substance can be administered once every two days or more. When administered systemically as an injection, the dose of the MMP12 inhibitory substance can also be administered once a day or in several doses. Conversely, the dose for several days can be administered in a single injection, whereby the MMP12 inhibitory substance can be administered once every two days or more. In addition, the MMP12 inhibitory substance may be administered continuously via drip infusion or the like. When locally administering a parenteral administration agent such as an ophthalmic preparation, a nasal drop, an ointment, a lotion, a cream, a gel, or a spray, the blending amount of the MMP12 inhibitory substance in the therapeutic agent as an external medicine, the frequency of local administration, and the area of application, can be adjusted as appropriate. In any case, administration does not necessarily have to be continuously or regularly performed, and can be performed at appropriate intervals in response to changes in symptoms. If a single dose causes cure or remission, there is no need to administer the therapeutic agent multiple times. If symptoms recur or worsen, administration may be resumed.

The method for administering the therapeutic agent of the present embodiment is not particularly limited, and since vasculitis syndrome, which is typically exemplified as a disease to be treated, causes inflammation of blood vessels, systemic administration by intravenous injection or drip infusion is preferred. When using an anti-MMP12 antibody or an aptamer as the MMP12 inhibitory substance, it is considered difficult to deliver a therapeutically effective dose to the affected area by oral administration, and thus parenteral administration is mainly selected. The administration is not limited to the administration by intravenous injection or drip infusion, and local administration such as intramuscular injection or the like may be performed. The administration period can be adjusted as appropriate according to the condition of the patient. The dosage during the administration period can be adjusted as appropriate, and examples thereof include continuously administering a constant amount or administering a relatively high dose initially and then switching to constant administration of a smaller maintenance dose.

In the therapeutic agent containing the MMP12 inhibitory substance according to the present embodiment, components that contribute to treatment for immune-mediated inflammatory diseases characterized by an increase in expression of MMP12, and another additive can also be used in combination independently of the MMP12 inhibitory substance. The components used in combination with the MMP12 inhibitory substance may be administered at the same time as or a different time from the MMP12 inhibitory substance, and the administration routes of both may be the same or different from each other.

The components used in combination can be purchased commercially or obtained or prepared using a method described in literature. The appropriate dosage of each component can also be determined as appropriate based on known information.

The present invention includes embodiments described below.

Item 1. A method for detecting an immune-mediated inflammatory disease characterized by an increase in expression of MMP12, in a subject, the method including
measuring a level of MMP12 in a biological sample obtained from the subject.

Item 2. The method according to Item 1, wherein the measuring the level of MMP12 is measuring a level of an MMP12 protein.

Item 3. The method according to Item 1, further including bringing the biological sample obtained from the subject into contact with a substance that binds to MMP12, wherein
the measuring the level of MMP12 includes measuring a level of a complex of MMP12 and a substance that binds to MMP12.

Item 4. The method according to Item 1, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 5. The method according to Item 1, wherein the biological sample is blood, serum, or plasma.

Item 6. The method according to Item 1, wherein the immune-mediated inflammatory disease characterized by an increase in expression of MMP12 is Takayasu arteritis.

Item 7. The method according to Item 1, further including bringing the biological sample obtained from the subject into contact with a peptide having a site that can be cleaved by MMP12, wherein
the measuring the level of MMP12 includes measuring a change caused by cleavage of the peptide having a site that can be cleaved by MMP12.

Item 8. The method according to any one of Items 1 to 7, wherein
the method is a method for predicting a possibility of developing the immune-mediated inflammatory disease characterized by an increase in expression of MMP12, in a subject,
the method further includes comparing the level of MMP12 in the biological sample obtained from the subject with a reference value, and
if the level of MMP12 in the biological sample obtained from the subject is higher than the reference value, it indicates that there is a high possibility that the subject will develop the immune-mediated inflammatory disease.

Item 9. The method according to any one of Items 1 to 7, wherein
the method is a method for predicting prognosis of a subject with the immune-mediated inflammatory disease characterized by an increase in expression of MMP12,
the subject is a subject who has received treatment for the immune-mediated inflammatory disease characterized by an increase in expression of MMP12,
the method further includes comparing the level of MMP12 in the biological sample obtained from the subject with a reference value, and
if the level of MMP12 in the biological sample obtained from the subject is higher than the reference value, it indicates that there is a high possibility of relapse of the immune-mediated inflammatory disease characterized by an increase in expression of MMP12, in the subject.

Item 10. The method according to any one of Items 1 to 7, wherein
the method is a method for predicting a post-treatment condition of a subject with the immune-mediated inflammatory disease characterized by an increase in expression of MMP12,
the subject is a subject who has received treatment for the immune-mediated inflammatory disease characterized by an increase in expression of MMP12,
the method further includes comparing the level of MMP12 in the biological sample obtained from the subject with a reference value, and
if the level of MMP12 in the biological sample obtained from the subject is lower than the reference value, it indicates that there is a high possibility that the immune-mediated inflammatory disease characterized by an increase in expression of MMP12, in the subject, has remitted.

Item 11. The method according to any one of Items 1 to 7, wherein
the method is a method for evaluating timing of administration of a therapeutic drug for the immune-mediated inflammatory disease characterized by an increase in expression of MMP12, to a subject with the immune-mediated inflammatory disease characterized by an increase in expression of MMP12,
the method further includes comparing the level of MMP12 in the biological sample obtained from the subject with a reference value, and
a fact that the level of MMP12 in the biological sample obtained from the subject is lower than the reference value is an indicator for discontinuing the administration of the drug to the subject.

Item 12. A diagnostic biomarker for an immune-mediated inflammatory disease characterized by an increase in expression of MMP12, including MMP12.

Item 13. The biomarker according to Item 12, including an MMP12 protein.

Item 14. A diagnostic drug for an immune-mediated inflammatory disease characterized by an increase in expression of MMP12, containing a substance that specifically interacts with MMP12.

Item 15. The diagnostic drug according to Item 14, wherein the diagnostic drug is an in vivo diagnostic drug including a conjugate of a peptide having a site that can be cleaved by MMP12 and a label substance.

Item 16. The diagnostic drug according to Item 15, wherein
the substance that specifically interacts with MMP12 is a peptide having a site that can be cleaved by MMP12, and
the conjugate includes a conjugate including a peptide having a site that can be cleaved by MMP12, a fluorophore bound to one end of the peptide, and a quencher bound to another end of the peptide.

Item 17. The diagnostic drug according to Item 15, wherein the label substance includes a fluorescent dye having a fluorophore, and the conjugate includes a conjugate including a peptide having a site that can be cleaved by MMP12, a fluorophore bound to one end of the peptide, and a quencher bound to another end of the peptide.

Item 18. The diagnostic drug according to any one of Items 14 to 17, wherein the peptide having a site that can be cleaved by MMP12 is either (i) or (ii) below:
(i) a peptide having any of partial sequences of amino acid sequences of human CXCL1 (UniPlot P09341), human CXCL2 (UniPlot P19875), human CXCL3 (UniPlot P19876), human CXCL5 (UniPlot P42830), human CXCL8 (UniPlot P10145), mouse CXCL1 (UniPlot A2RTH0), mouse CXCL2 (UniPlot P12850), mouse CXCL3 (UniPlot P10889), and mouse CXCL5 (UniPlot P50228), having an amino acid sequence of ELRCXC (X is Q or V), having a sequence of 1 to 5 amino acids upstream thereof, having a sequence of one or more amino acids downstream thereof, and composed of a sequence of 8 to 25 amino acids as a total length; and
(ii) a peptide in which, in the peptide of (i) above, further, one or more of amino acids upstream of the amino acid sequence of ELRCXC (X is Q or V) are substituted, one or more of amino acids downstream of the amino acid sequence of ELRCXC (X is Q or V) are substituted, or one or more of the amino acids upstream of the amino acid sequence of ELRCXC (X is Q or V) are substituted and one or more of the amino acids downstream of the amino acid sequence of ELRCXC (X is Q or V) are substituted.

Item 19. The diagnostic drug according to any one of Items 14 to 17, wherein the peptide having a site that can be cleaved by MMP12 is any of (i) to (vi) below:
(i) a peptide having any of amino acid sequences of SEQ ID NOs: 1 to 9;
(ii) a peptide composed only of any of the amino acid sequences of SEQ ID NOs: 1 to 9;
(iii) a peptide composed of a partial sequence of a peptide having any of the amino acid sequences of SEQ ID NOs: 1 to 9 having a part of ELRCXC (X is Q or V); and
(iv) a peptide in which, in the peptides of (i) to (iii) above, further, one or more of the first to fifth amino acids of any of the amino acid sequences of SEQ ID NOs: 1 to 9 are substituted, one or more of the 12th and 13th amino acids of any of the amino acid sequences of SEQ ID NOs: 1 to 9 are substituted, or both one or more of the first to fifth amino acids of any of the amino acid sequences of SEQ ID NOs: 1 to 9 and one or more of the 12th and 13th amino acids of any of the amino acid sequences of SEQ ID NOs: 1 to 9 are substituted.

Item 20. The diagnostic drug according to any one of Items 14 to 17, wherein the peptide having a site that can be cleaved by MMP12 is any of (i) to (v) below:
(i) a peptide having any of amino acid sequences of SEQ ID NOs: 10 to 32;
(ii) a peptide composed only of any of the amino acid sequences of SEQ ID NOs: 10 to 32;
(iii) a peptide composed of a partial sequence of a peptide having any of the amino acid sequences of SEQ ID NOs: 10 to 32 having a site that is cleaved by MMP12, and composed of a sequence of 8 to 25 amino acids as a total length;
(iv) a peptide in which, in any of the peptides of (i) to (iii) above, one or more of amino acids other than amino acids having a site that is cleaved by MMP12 are substituted; and
(v) a peptide in which, in any of the peptides of (i) to (iii) above, all cysteines in a natural sequence are substituted with other amino acids such as serine.

Item 21. The diagnostic drug according to any one of Items 14 to 17, wherein the peptide having a site that can be cleaved by MMP12 is any of (i) to (v) below:
(i) a peptide having any of amino acid sequences of SEQ ID NOs: 11, 13, 14, 15, 16, 20, 24, 25, 26, 27, 29, and 31;
(ii) a peptide composed only of any of the amino acid sequences of SEQ ID NOs: 111, 13, 14, 15, 16, 20, 24, 25, 26, 27, 29, and 31;
(iii) a peptide composed of a partial sequence of a peptide having any of the amino acid sequences of SEQ ID NOs: 111, 13, 14, 15, 16, 20, 24, 25, 26, 27, 29, and 31 having a site that is cleaved by MMP12, and composed of a sequence of 8 to 25 amino acids as a total length;
(iv) a peptide in which, in any of the peptides of (i) to (iii) above, one or more of amino acids other than amino acids having a site that is cleaved by MMP12 are substituted; and
(v) a peptide in which, in any of the peptides of (i) to (iii) above, all cysteines in a natural sequence are substituted with other amino acids such as serine.

Item 22. The diagnostic drug according to any one of Items 14 to 21, wherein the peptide having a site that can be cleaved by MMP12 is a peptide composed of a sequence of 8 to 25 amino acids.

Item 23. The diagnostic drug according to any one of Items 14 to 21, wherein the peptide having a site that can be cleaved by MMP12 is a peptide composed of a sequence of 10 to 20 amino acids.

Item 24. The diagnostic drug according to any one of Items 14 to 21, wherein the peptide having a site that can be cleaved by MMP12 is a peptide composed of a sequence of 12 to 18 amino acids.

Item 25. The diagnostic drug according to Item 15 or 16, wherein the label substance includes a fluorescent dye having a fluorophore, and the fluorescent dye is at least one fluorescent dye selected from among phenyl and derivatives thereof, naphthalene and derivatives thereof such as 5-dimethylaminonaphthalene-1-sulfonic acid and hydroxynaphthalenes, anthracene and derivatives thereof such as 9,10-diphenylnaphthalene and 9-methylanthracene, pyrene and derivatives thereof such as N-(1-pyrene)iodoacetamide and hydroxypyrenes, biphenyl and derivatives thereof, acridine and derivatives thereof such as hydroxyacridines and 9-methylacridine, coumarin and derivatives thereof such as 7-dialkylamino-4-methylcoumarin and 4-bromomethyl-7-methoxycoumarin, xanthene and derivatives thereof, phthalocyanine and derivatives thereof, stilbene and derivatives thereof such as 6,6'-dibromostilbene and hydroxystilbenes, furan and derivatives thereof, oxazole and derivatives thereof, oxadiazole and derivatives thereof, nitrobenzoxadiazole and derivatives thereof such as hydroxynitrobenzoxadiazoles, benzothiazole and derivatives thereof, fluorescein and derivatives thereof such as 5-iodoacetamidofluorescein and fluorescein-5-maleimide, rhodamine and derivatives thereof such as tetramethylrhodamine, tetraethylrhodamine, and carboxy tetramethylrhodamine, BODIPY (registered trademark) and derivatives thereof, eosin and derivatives thereof, erythrosine and derivatives thereof such as hydroxyerythrosines and 5-iodoacetamidoerythrosine, resorufin and derivatives thereof such as hydroxyresorufin, quinoline and derivatives thereof such as 6-hydroxyquinoline and 6-aminoquinoline, carbazole and derivatives thereof such as N-methylcarbazole, cyanine and derivatives thereof such as hydroxycyanine, carbocyanine and derivatives such as phenylcarbocyanine, pyridinium salts and derivatives thereof such as 4-(4-dialkyldiamidostyryl)-N-methylpyridinium iodide, fluorescent complexes of lanthanide and derivatives thereof, green fluorescent protein (GFP) and derivatives thereof, and benzoindole and derivatives thereof (such as indocyanine green).

Item 26. The diagnostic drug according to Item 15 or 16, wherein the label substance includes a fluorescent dye having a fluorophore, and the fluorescent dye is at least one fluorescent dye selected from among a fluorescein and derivatives thereof, rhodamine and derivatives thereof, BODIPY (registered trademark) and derivatives thereof, and cyanine and derivatives thereof.

Item 27. The diagnostic drug according to any one of Items 14 to 26, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 28. A diagnostic kit for diagnosing an immune-mediated inflammatory disease characterized by an increase in expression of MMP12, the diagnostic kit including a substance that specifically interacts with MMP12.

Item 29. The diagnostic kit according to Item 28, wherein the substance that specifically interacts with MMP12 is a substance that binds to MMP12, selected from the group consisting of an antibody to MMP12, an antigen-binding fragment of an antibody to MMP12, and an aptamer to MMP12.

Item 30. The diagnostic kit according to Item 28, wherein the substance that specifically interacts with MMP12 includes a peptide having a site that can be cleaved by MMP12.

Item 31. The diagnostic kit according to Item 28, wherein a label substance is bound to the peptide having a site that can be cleaved by MP12.

Item 32. The diagnostic kit according to Item 26, wherein a fluorophore is bound to one end of the peptide having a site that can be cleaved by MP12, and a quencher is bound to another end of the peptide.

Item 33. The diagnostic kit according to any one of Items 30 to 32, wherein the peptide having a site that can be cleaved by MMP12 is either (i) or (ii) below:
(i) a peptide having any of partial sequences of amino acid sequences of human CXCL1 (UniPlot P09341), human CXCL2 (UniPlot P19875), human CXCL3 (UniPlot P19876), human CXCL5 (UniPlot P42830), human CXCL8 (UniPlot P10145), mouse CXCL1 (UniPlot A2RTH0), mouse CXCL2 (UniPlot P12850), mouse CXCL3 (UniPlot P10889), and mouse CXCL5 (UniPlot P50228), having an amino acid sequence of ELRCXC (X is Q or V), having a sequence of 1 to 5 amino acids upstream thereof, having a sequence of one or more amino acids downstream thereof, and composed of a sequence of 8 to 25 amino acids as a total length; and
(ii) a peptide in which, in the peptide of (i) above, further, one or more of amino acids upstream of the amino acid sequence of ELRCXC (X is Q or V) are substituted, one or more of amino acids downstream of the amino acid sequence of ELRCXC (X is Q or V) are substituted, or one or more of the amino acids upstream of the amino acid sequence of ELRCXC (X is Q or V) are substituted and one or more of the amino acids downstream of the amino acid sequence of ELRCXC (X is Q or V) are substituted.

Item 34. The diagnostic kit according to any one of Items 30 to 32, wherein the peptide having a site that can be cleaved by MMP12 is any of (i) to (vi) below:
(i) a peptide having any of amino acid sequences of SEQ ID NOs: 1 to 9;
(ii) a peptide composed only of any of the amino acid sequences of SEQ ID NOs: 1 to 9;
(iii) a peptide composed of a partial sequence of a peptide having any of the amino acid sequences of SEQ ID NOs: 1 to 9 having a part of ELRCXC (X is Q or V); and
(iv) a peptide in which, in the peptides of (i) to (iii) above, further, one or more of the first to fifth amino acids of any of the amino acid sequences of SEQ ID NOs: 1 to 9 are substituted, one or more of the 12th and 13th amino acids of any of the amino acid sequences of SEQ ID NOs: 1 to 9 are substituted, or both one or more of the first to fifth amino acids of any of the amino acid sequences of SEQ ID NOs: 1 to 9 and one or more of the 12th and 13th amino acids of any of the amino acid sequences of SEQ ID NOs: 1 to 9 are substituted.

Item 35. The diagnostic kit according to any one of Items 30 to 32, wherein the peptide having a site that can be cleaved by MMP12 is any of (i) to (v) below:
(i) a peptide having any of amino acid sequences of SEQ ID NOs: 10 to 32;
(ii) a peptide composed only of any of the amino acid sequences of SEQ ID NOs: 10 to 32;
(iii) a peptide composed of a partial sequence of a peptide having any of the amino acid sequences of SEQ ID NOs: 10 to 32 having a site that is cleaved by MMP12, and composed of a sequence of 8 to 25 amino acids as a total length;
(iv) a peptide in which, in any of the peptides of (i) to (iii) above, one or more of amino acids other than amino acids having a site that is cleaved by MMP12 are substituted; and
(v) a peptide in which, in any of the peptides of (i) to (iii) above, all cysteines in a natural sequence are substituted with other amino acids such as serine.

Item 36. The diagnostic kit according to any one of Items 30 to 32, wherein the peptide having a site that can be cleaved by MMP12 is any of (i) to (v) below:
(i) a peptide having any of amino acid sequences of SEQ ID NOs: 11, 13, 14, 15, 16, 20, 24, 25, 26, 27, 29, and 31;
(ii) a peptide composed only of any of the amino acid sequences of SEQ ID NOs: 111, 13, 14, 15, 16, 20, 24, 25, 26, 27, 29, and 31;
(iii) a peptide composed of a partial sequence of a peptide having any of the amino acid sequences of SEQ ID NOs: 111, 13, 14, 15, 16, 20, 24, 25, 26, 27, 29, and 31 having a site that is cleaved by MMP12, and composed of a sequence of 8 to 25 amino acids as a total length;
(iv) a peptide in which, in any of the peptides of (i) to (iii) above, one or more of amino acids other than amino acids having a site that is cleaved by MMP12 are substituted; and
(v) a peptide in which, in any of the peptides of (i) to (iii) above, all cysteines in a natural sequence are substituted with other amino acids such as serine.

Item 37. The diagnostic kit according to any one of Items 30 to 37, wherein the peptide having a site that can be cleaved by MMP12 is a peptide composed of a sequence of 8 to 25 amino acids.

Item 38. The diagnostic kit according to any one of Items 30 to 37, wherein the peptide having a site that can be cleaved by MMP12 is a peptide composed of a sequence of 10 to 20 amino acids.

Item 39. The diagnostic kit according to any one of Items 30 to 37, wherein the peptide having a site that can be cleaved by MMP12 is a peptide composed of a sequence of 12 to 18 amino acids.

Item 40. The diagnostic kit according to Item 31, wherein the label substance includes a fluorescent dye having a fluorophore, and the fluorescent dye is at least one fluorescent dye selected from among phenyl and derivatives thereof, naphthalene and derivatives thereof such as 5-dimethylaminonaphthalene-1-sulfonic acid and hydroxynaphthalenes, anthracene and derivatives thereof such as 9,10-diphenylnaphthalene and 9-methylanthracene, pyrene and derivatives thereof such as N-(1-pyrene)iodoacetamide and hydroxypyrenes, biphenyl and derivatives thereof, acridine and derivatives thereof such as hydroxyacridines and 9-methylacridine, coumarin and derivatives thereof such as 7-dialkylamino-4-methylcoumarin and 4-bromomethyl-7-methoxycoumarin, xanthene and derivatives thereof, phthalocyanine and derivatives thereof, stilbene and derivatives thereof such as 6,6'-dibromostilbene and hydroxystilbenes, furan and derivatives thereof, oxazole and derivatives thereof, oxadiazole and derivatives thereof, nitrobenzoxadiazole and derivatives thereof such as hydroxynitrobenzoxadiazoles, benzothiazole and derivatives thereof, fluorescein and derivatives thereof such as 5-iodoacetamidofluorescein and fluorescein-5-maleimide, rhodamine and derivatives thereof such as tetramethylrhodamine, tetraethylrhodamine, and carboxy tetramethylrhodamine, BODIPY (registered trademark) and derivatives thereof, eosin and derivatives thereof, erythrosine and derivatives thereof such as hydroxyerythrosines and 5-iodoacetamidoerythrosine, resorufin and derivatives thereof such as hydroxyresorufin, quinoline and derivatives thereof such as 6-hydroxyquinoline and 6-aminoquinoline, carbazole and derivatives thereof such as N-methylcarbazole, cyanine and derivatives thereof such as hydroxycyanine, carbocyanine and derivatives such as phenylcarbocyanine, pyridinium salts and derivatives thereof such as 4-(4-dialkyldiamidostyryl)-N-methylpyridinium iodide, fluorescent complexes of lanthanide and derivatives thereof, green fluorescent protein (GFP) and derivatives thereof, and benzoindole and derivatives thereof (such as indocyanine green).

Item 41. The diagnostic kit according to Item 31, wherein the label substance includes a fluorescent dye having a fluorophore, and the fluorescent dye is at least one fluorescent dye selected from among a fluorescein and derivatives thereof, rhodamine and derivatives thereof, BODIPY (registered trademark) and derivatives thereof, and cyanine and derivatives thereof.

Item 42. The diagnostic kit according to any one of Items 28 to 41, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 43. A method for diagnosing an immune-mediated inflammatory disease characterized by an increase in expression of MMP12, in a subject, using the diagnostic drug according to any one of Items 14 to 27 or the diagnostic kit according to any one of Items 28 to 42.

Item 44. A method for screening for a substance effective for treatment for an immune-mediated inflammatory disease characterized by an increase in expression of MMP12, the method including:
measuring a level of MMP12 in a biological sample obtained from a subject after administration of a test substance; and
if the level of MMP12 is decreased due to the administration of the test substance, selecting the test substance as a candidate substance effective for treatment for the immune-mediated inflammatory disease characterized by an increase in expression of MMP12.

Item 45. The method according to Item 44, wherein the measuring the level of MMP12 includes measuring a level of a complex of MMP12 and a substance that binds to MMP12.

Item 46. The method according to Item 45, wherein the substance that binds to MMP12 is selected from the group consisting of an antibody to MMP12, an antigen-binding fragment of an antibody to MMP12, and an aptamer to MMP12.

Item 47. The method according to any one of Items 44 to 46, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 48. The method according to any one of Items 44 to 47, wherein the biological sample is blood, serum, or plasma.

Item 49. A therapeutic agent for an immune-mediated inflammatory disease characterized by an increase in expression of MMP12, containing an MMP12 inhibitory substance.

Item 50. The therapeutic agent according to Item 49, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 51. The therapeutic agent according to Item 49, wherein the immune-mediated inflammatory disease characterized by an increase in expression of MMP12 includes ANCA-associated vasculitis.

Item 52. A method for treating an immune-mediated inflammatory disease characterized by an increase in expression of MMP12, in a subject in need of treatment for the immune-mediated inflammatory disease, the method including administering an MMP12 inhibitory substance to the subject in an amount effective for treatment.

Item 53. The method according to Item 52, wherein the immune-mediated inflammatory disease characterized by an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

Item 54. The method according to Item 52, wherein the immune-mediated inflammatory disease characterized by an increase in expression of MMP12 includes vasculitis or granulomatous vasculitis.

The disclosures of all patent applications and literatures cited herein are incorporated herein by reference in their entirety.

The present invention is more specifically described by way of Examples below, but the present invention is not limited to these Examples.

### Examples

### Example 1: Identification of Vasculitis-Specific Molecules

As screening for vasculitis-specific molecules using serum proteomes, the Inflammation panel, Immune response panel, Cardiovascular II panel, and Cardiovascular III panel of the Olink proximity extension assay (Uppsala), which detects and quantifies double-stranded DNA sequences obtained through hybridization by binding two antibodies having oligo DNA added thereto to a single target antigen protein, are used. Of 368 types of proteins measured, 354 types of proteins were evaluated after excluding duplicates. Patients with Giant cell arteritis (GCA), Takayasu arteritis (TAK), polyarteritis nodosa (PAN), granulomatosis with polyangiitis (GPA), eosinophilic granulomatosis with polyangiitis (EGPA), microscopic polyangiitis (MPA), Behcet disease (BD), relapsing polychondritis (RP), and adult onset still disease (AOSD), and healthy subjects were subjects. The patients were classified into five groups (1: GCA, TAK, PAN, GPA, EGPA, MPA; 2: AOSD, BD, RP; 3: PMR, RA, SpA; 4: SLE, SSc, IIM; 5: IgG4RD, SjS) based on clinical features, and proteins that were significantly increased in each group compared to the healthy subjects, as a result of multiple correction tests, were identified. The identified proteins were plotted on a Venn diagram, and molecules specific to vasculitis (Group 1) were identified.

### (Results)

As molecules specific to each group, Group 1: 6; Group 2: 17; Group 3: 0; Group 4: 9; and Group 5: 1 were identified. When ROC analysis was performed using the NPX values measured by Olink, with molecules 1-6 specific to vasculitis (Group 1) as a target, in order to compare the group of patients with vasculitis syndrome with the group of healthy subjects and compare the group of patients with vasculitis syndrome with the groups of patients with the immune-mediated inflammatory diseases other than vasculitis syndrome, it was found that MMP12 (solid line) had the highest sensitivity in terms of AUC determination (Fig. 1(A) and Fig. 1(B)). Validation was performed by ELISA (Thermo Fisher Scientific) using the same serum samples, and the results showed that the serum MMP12 values were characteristically increased in granulomatous vasculitis (GCA, EGPA, GPA, MPA, PAN, TAK), were also high values in IgG4 related disease (IgG4RD), inflammatory bowel disease, and nontuberculous mycobacterial infection(NTM) (Fig. 2), and were also high values in some of the patients with polymyalgia rheumatica (PMR), rheumatoid arthritis (RA), and systemic lupus erythematosus (SLE). In the immune-mediated inflammatory diseases as the control group, no increase in expression of MMP12 was observed, which was useful for differentiating from these diseases. Furthermore, MMP12 was normal in patients with general bacterial and viral infections, so that it was also possible to use MMP12 to distinguish between these infections and vasculitis syndrome.

### Example 2: Expression of MMP12 in Tissues

MMP12 is not expressed in peripheral blood immune cells. To clarify whether MMP12 is derived from tissues, immunohistochemical staining of MMP12 was performed on vasculitis tissues of Takayasu arteritis (TAK), giant cell arteritis (GCA), and granulomatosis with polyangiitis (GPA), which are immune-mediated inflammatory diseases characterized by an increase in expression of MMP12. MMP12 was stained using Goat anti-human MMP12 (R&D) and compared with HE staining of serial sections and immunohistochemical staining of CD68 and CD206.

### (Results)

As a result of immunohistochemical staining, expression of MMP12 was observed in the aortae of patients with Takayasu arteritis (Fig. 3), the temporal arteries of patients with giant cell arteritis (Fig. 4), and the lungs of patients with polyangiitis granulomatosis (Fig. 5). In comparison with HE staining, expression of MMP12 was observed in histiocytes and multinucleated giant cells. In comparison with histochemical staining, MMP12-positive cells were CD68-positive and CD206-positive, so that it was confirmed that MMP12 was derived from tissue macrophages in vasculitis. It was found that alveolar macrophages are CD68-positive and CD206-positive but MMP12-negative, and an MMP12-positive phenotype exists in some of CD206-positive macrophages.

### Example 3: Correlation with PET-CT Score (PETVAS)

MMP12 was measured by ELISA (Thermo Fisher Scientific) using serum samples taken before treatment for Takayasu arteritis and giant cell arteritis. Since the PET vascular activity score (PETVAS) which is used in PET-CT examinations is known to be associated with a relapse rate which is an indicator for the treatment prognosis for Takayasu arteritis and giant cell arteritis (Arthritis Rheumatol 2018; 70: 439-449.), the correlation between serum MMP12 value and PETVAS was examined with patients from which both were obtained, as subjects. In this example, a cutoff value of 20 or higher, which has been reported to be associated with a high relapse rate, was set for PETVAS, and a value that separates a group of healthy subjects and a group of patients diagnosed as having the immune-mediated inflammatory diseases characterized by an increase in expression of MMP12 based on a doctor's diagnosis (100 pg/ml) and a certain value that is higher than an average calculated from measurement of the levels of MMP12 in a plurality of healthy subjects and is lower than an average calculated from measurement of the levels of MMP12 in a plurality of patients with the immune-mediated inflammatory diseases characterized by an increase in expression of MMP12 (500 pg/ml) were set as cut-off values for serum MMP12.

### (Results)

MMP12 at the time of diagnosis of Takayasu arteritis and giant cell arteritis was positively correlated with PETVAS (Fig. 6). MMP12 was related to the relapse rate, which is a treatment prognosis indicator, regardless of whether PETVAS was a high value (≥20) or a low value (<20) (Fig. 7), and it was found that if serum MMP12 was a high value, the prognosis was poor. The method of the present embodiment can be a better evaluation method than PETVAS and is useful for stratifying the risk of relapse in a subject.

### Example 4: Evaluation of Remission Using MMP12 as Indicator

MMP12 was measured by ELISA (Thermo Fisher Scientific) using serum samples taken before treatment and during treatment for Takayasu arteritis and giant cell arteritis. Patients who maintained remission without relapse (patients 1-19; Takayasu arteritis: n=9; giant cell arteritis: n=10) were subjects, and the serum MMP12 values measured over time were plotted. The transition of the serum MMP12 value and the transition in imaging tests such as contrast CT, contrast MRI, and PET-CT, which are indicators for the activity of vasculitis, were compared.

### (Results)

Of the patients 1-19 with Takayasu arteritis and giant cell arteritis who maintained remission without relapse, the patients 1-11 exhibited a monotonous descending type in which MMP12 was normalized with treatment and was maintained at normal levels (Fig. 8(A)). The patients 12-19 exhibited a smoldering type in which MMP12 was not normalized or was normalized and then increased again (Fig. 9(A)). In the monotonous descending type, disease activity, such as vessel wall thickening, which was observed in the imaging tests, disappeared, so that MMP12 was useful as a criterion for determining remission (Figs. 8(B) and (C)). On the other hand, in the smoldering type, the vessel wall thickening continued, reflecting latent disease activity (Figs. 9(B) and (C)). The monotonous descending type (n=11) was Takayasu arteritis (n=3) and giant cell arteritis (n=9), and the smoldering type (n=8) was Takayasu arteritis (n=6) and giant cell arteritis (n=2). Of the smoldering type of Takayasu arteritis (n=6), two patients had inflammatory bowel disease, and MMP12 was kept at a high value in Takayasu arteritis, especially in Takayasu arteritis accompanying inflammatory bowel disease, which suggested that it may be difficult to lead to remission.

### Example 5: Evaluation of Drug Withdrawal Using MMP12 as Indicator

Of the patients 1-19 with Takayasu arteritis and giant cell arteritis who maintained remission without relapse, the patient 19 maintained drug-free remission. MMP12 was measured by ELISA (Thermo Fisher Scientific) using serum samples taken before treatment and during treatment from the patient who maintained drug-free remission.

### (Results)

After discontinuation of steroids and tocilizumab (Actemra (registered trademark)), a transient increase in serum MMP12 was observed, but MMP12 subsequently decreased and was maintained at normal levels (<100 pg/ml). It may be possible to use normal MMP12 levels as an indicator for drug withdrawal.

### Example 6: Evaluation of Relapse under Immunosuppressive Treatment with IL-6 Receptor Inhibitors.

MMP12 was measured by ELISA (Thermo Fisher Scientific) using serum samples taken before treatment and during treatment for Takayasu arteritis and giant cell arteritis. Patients who experienced relapse within two years from the start of treatment (patients 20-32; Takayasu arteritis: n=4, giant cell arteritis: n=9) were subjects, and the serum MMP12 values measured over time were plotted. The transition of the serum MMP12 value and the transition in imaging tests such as contrast CT, contrast MRI, and PET-CT, which are indicators for the activity of vasculitis, were compared.

### (Results)

Of the patients 20-31 with Takayasu arteritis and giant cell arteritis who experienced relapse, the serum MMP12 value was normalized during the remission period and increased at the time of relapse in all the cases. Furthermore, in 5 of the 6 cases where samples were obtained prior to relapse, an increase in MMP12 prior to determination of relapse based on clinical symptoms and imaging tests was observed (Fig. 10(A)). The vessel wall thickening that had disappeared during the remission period was observed again at the time of relapse (Figs. 10(B) to (D)).

Of the patients 20-31 with Takayasu arteritis and giant cell arteritis who experienced relapse, 7 patients experienced relapse while using tocilizumab (Actemra (registered trademark)), but MMP12 reflected the disease activity before and after the relapse, as in the 5 patients who experienced relapse while not using tocilizumab (Actemra (registered trademark)).

### Example 7: Therapeutic Effects of MMP12 Inhibitors on ANCA-Associated Vasculitis Model Mice

SCG/ThpNkc mice (see Proc Natl Acad Sci U S A. 1993 Apr 15; 90(8): 3413-7.) are ANCA-associated vasculitis model mice created by crossing and interbreeding two strains, MRL/lpr and BXSB, and can be supplied from Laboratory Animal Resource Bank at the National Institute of Biomedical Innovation, Health and Nutrition (resource number: nbio133).

In this experiment, MMP408 (CAS No. 1258003-93-8), which is an MMP12 selective inhibitor, was administered to male SCG/ThpNkc mice, and the therapeutic effects of MMP408 were examined.

The SCG/ThpNkc mice were reared in cages with normal solid food and water. At 10 weeks of age, it was confirmed that the mice had positive occult blood in their urine. From 11 weeks of age, MMP408 was administered via the intraperitoneal route at a dose of 100 µg twice a week. Urine analysis was performed at 15 and 20 weeks of age, and the mice were sacrificed at 20 weeks of age. The number of mice in an MMP408 non-administration group and the number of mice in an MMP408 administration group were n=25 and n=4, respectively.

SCG/ThpNkc mice die at 10-20 weeks, which is close to the clinical picture of ANCA-associated vasculitis, and thus are suitable for drug screening using a survival curve, but as shown in Fig. 11, while 92% of the mice in the MMP408 non-administration group died by 20 weeks, 2 out of 4 mice were still alive at 20 weeks in the MMP408 administration group (log-rank test p=0.037). In addition, as shown in Fig. 12, the occult blood also improved at week 20.

### Example 8: Development of Probe for Detecting MMP12

### 1. Selection of peptides

Substrate peptides that were highly selective for MMP12 were searched for as components of a probe for detecting MMP12.

First, in order to create the probe for detecting MMP12, the areas around the cleavage sites of known proteins that are known to be cleaved by MMP12 were investigated for the peptide portion, and candidate peptide Nos. 1 to 23 were selected (Figs. 13 and 14, Tables 1 and 2). PP-2b and PJ-8 are positive controls, and the arrows in Figs. 13 and 14 indicate the cleavage sites by MMP12. The following 1) to 4) were taken into account when selecting peptides. 1) The introduction position of the fluorescent dye was set to the N-terminal side. 2) Glycine (Gly) was introduced as a spacer to reduce the effect of the fluorescent dye on enzyme recognition. 3) The secondary structure near the cleavage site of the peptide was included in consideration of the possibility that the secondary structure was involved in recognition by the MMP12 enzyme. 4) The length of amino acids was set to about 15 residues to facilitate peptide synthesis.

**[Table 1]**

| | | | | | |
|---|---|---|---|---|---|
| 1 | A1 | hCCL-14 | 1-12 | N-terminal sequence | Delete Pro |
| 2 | B0 | | 61-74 | C-terminal sequence (a helix) | - |
| 3 | C1 | hIFN-α-10 | 154-165 | C-terminal sequence (a helix on N side) | Includes C-side cleavage sequence |
| 4 | C2 | | 150-161 | | Does not include C-side cleavage sequence |
| 5 | D1 | hIFN-α-6 | 154-165 | C-terminal sequence (a helix on N side) | Includes C-side cleavage sequence |
| 6 | D2 | | 150-161 | | Does not include C-side cleavage sequence |
| 7 | E1 | hIFN-α-2 | 154-165 | C-terminal sequence (a helix on N side) | Includes C-side cleavage sequence |
| 8 | E2 | | 150-161 | | Does not include C-side cleavage sequence |
| 9 | F1 | hIFN-α-8 | 154-165 | C-terminal sequence (a helix on N side) | Includes C-side cleavage sequence |
| 10 | F2 | | 150-161 | | Does not include C-side cleavage sequence |

**[Table 2]**

| | | | | | |
|---|---|---|---|---|---|
| 11 | G1 | hIFN-γ | 126-139 | Inside from C-terminal (a helix) | Includes N-side helix |
| 12 | G2 | | 130-143 | | Includes C-side helix |
| 13 | H1 | | 151-165 | C-terminal sequence | Predicted to have no secondary structure |
| 14 | J1 | mIFN-γ | 125-137 | Inside from C-terminal (a helix) | Includes N-side helix |
| 15 | J2 | | 128-141 | | Includes C-side helix |
| 16 | K1 | | 143-154 | C-terminal sequence | Delete Cys |
| 17 | L1 | mCXCL-1 | 1-14 | N-terminal sequence (SS bond on C side) | Replace Cys with Ser |
| 18 | M1 | mCXCL-5 | 78-90 | C-terminal sequence (a helix) | Delete N-side helix |
| 19 | N1 | hCXCL-7 | 76-89 | C-terminal sequence (a helix) | Delete Pro-Asp at N-terminal |
| 20 | P1 | hCXCL-3 | 1-14 | N-terminal sequence (SS bond on C side) | Replace Cys with Ser |
| 21 | Q2 | hCXCL-8 | 3-14 | N-terminal sequence (SS bond on C side) | Replace Cys with Ser Delete C-terminal |
| 22 | R2 | hCXCL-5 | 3-15 | N-terminal sequence (SS bond on C side) | Replace Cys with Ser Delete C-terminal |
| 23 | S2 | hCXCL-6 | 9-22 | N-terminal sequence (SS bond on C side) | Replace Cys with Ser Delete N-terminal |

### 2. Synthesis of Fluorescent Labeled Peptide

First, a peptide was synthesized using the Biotage (registered trademark) synthesizer (resin: MBHA, condensing agent: DIC/Oxyma, washing agent: DMF, 5 equivalents of amino acid). Next, the peptide was modified with 5-carboxy fluorescein (5-CF) (5-CF: 1.2 equivalents, condensing agent: DIC/Oxyma, washing agent: DMF/DCM, dried in a desiccator for 2 hours). Next, the peptide side chain was deprotected and the resin was removed from the peptide (using TFA/Water/TIS, washing agent: TFA). The product was roughly purified by ether precipitation and purified by HPLC, and the purity was confirmed by HPLC analysis. It was confirmed by mass spectrometry that the target peptide had been synthesized, and the synthesized peptide was subjected to cleavage evaluation described below.

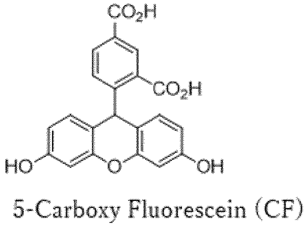

### 3. Evaluation of Cleavage of Candidate Peptides by MMP12

The evaluation of cleavage of candidate peptides by MMP12 was performed as follows.

On the first day, h MMP12 was diluted to 50 µg/mL with an assay buffer. APMA was added to the h MMP12 solution to adjust the final concentration of APMA to 1 mM, and the solution was incubated at 37°C for 24 hours to activate h MMP12.

On the second day, activated h MMP12 was diluted to 2 ng/µL (=40 nM) with an assay buffer. The substrate (peptide) was diluted to 40 µM with an assay buffer. 50 µL of h MMP12 (2.0 ng/µL) and 50 µL of the substrate (peptide) were mixed and incubated at 37°C for 16 hours. A blank was prepared by mixing equal volumes of an assay buffer and the substrate (peptide).

On the third day, 100 µL of acetonitrile was added to the mixture from the second day. After centrifugation for 10 minutes, the supernatant was collected, and 10 µL of the supernatant was analyzed using ultra high performance liquid chromatography (UPLC).

UPLC was performed under the conditions of measurement wavelength: 440 nm, flow rate: 0.4 mL/min, A: H₂O with 0.1% TFA, B: CH₃CN with 0.1% TFA, and elution conditions 10% B → 70% B in 9.5 min, and as a result, it was confirmed that the two positive controls were cleaved by MMP12 (data is not shown).

### 4. Evaluation of Cleavage of Each Candidate Peptide by MMP12

For the candidate peptide Nos. 2, 4 to 7, 11, 15 to 17, 19, 20, and 22, the evaluation of cleavage by MMP12 was performed in the same manner as for the positive controls. The results are shown in Table 3. Cleavage by MMP12 was confirmed for all the peptides tested. For Nos. 2 and 4 to 7, the cleavage sites were L-Q and L-R/K, but the cleavage rates were different. The cleavage rates of Nos. 2 and 4 were medium, and the cleavage rates of Nos. 5, 6, and 7, which have N-L-Q-E, were medium to high. For Nos. 11, 15, 17, 20, and 22, the cleavage site was E-L, but the cleavage rates were different. The cleavage rate of No. 11 was high and the cleavage rate of No. 15 was low even though these candidate peptides have E-L-I. The cleavage rates of Nos. 17, 20, and 22, which have E-L-R, were high.

**[Table 3]**

| Cleavage rate of each candidate peptide by MMP12 | | | | | | | | | | |
|---|---|---|---|---|---|---|---|---|---|---|
| Candidate sequence | Origin of sequence | | Number of cleavage peaks | | Retention time (min) | | | Peak area | | |
| | Substrate | aa. | Predict | Test | before | after | Δtime | before | after | Cleavage rate (%) |
| No. 02 | hCCL-14 | 61-74 | 2 | 1 | 2.69 | 3.01 | 0.32 | 68354 | 30048 | 56.0 |
| No. 04 | hIFN-α-10 | 150-161 | 1 | 1 | 2.98 | 2.80 | 0.17 | 81667 | 38008 | 53.5 |
| No. 05 | hIFN-α-6 | 154-165 | 2 | 1 | 2.15 | 2.09 | 0.07 | 28732 | 17680 | 38.5 |
| No. 06 | | 150-161 | 1 | 1 | 2.66 | 2.51 | 0.15 | 90676 | 8762 | 90.3 |
| No. 07 | hIFN-α-2 | 154-165 | 2 | 2 | 2.32 | 2.21 | 0.11 | 45510 | 13459 | 70.4 |
| No. 11 | hIFN-γ | 126-139 | 1 | 2 | 3.30 | 2.70 | 0.61 | 24295 | 0 | 100.0 |
| No. 15 | mIFN-γ | 128-141 | 1 | 1 | 4.24 | 2.47 | 1.77 | 37273 | 33359 | 10.5 |
| No. 16 | | 143-154 | 1 | 1 | 2.08 | 2.26 | 0.18 | 81257 | 31122 | 61.7 |
| No. 17 | mCXCL-1 | 1-14 | 2 | 1 | 3.00 | 2.63 | 0.37 | 82465 | 1141 | 98.6 |
| No. 19 | hCXCL-7 | 76-89 | 1 | 1 | 2.66 | 2.18 | 0.48 | 78156 | 57797 | 26.0 |
| No. 20 | hCXCL-3 | 1-14 | 1 | 2 | 2.98 | 2.60 | 0.38 | 102080 | 0 | 100.0 |
| No. 22 | hCXCL-5 | 3-15 | 1 | 3 | 4.13 | 3.24 | 0.89 | 57679 | 0 | 100.0 |

## Claims

1. A method for detecting an immune-mediated inflammatory disease **characterized by** an increase in expression of MMP12, in a subject, the method comprising
measuring a level of an MMP12 protein in a biological sample obtained from the subject.

2. The method according to claim 1, further comprising bringing the biological sample obtained from the subject into contact with a substance that binds to MMP12, wherein
the measuring the level of the MMP12 protein includes measuring a level of a complex of the MMP12 protein and a substance that binds to the MMP12 protein.

3. The method according to claim 1, wherein the immune-mediated inflammatory disease **characterized by** an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

4. The method according to claim 1, wherein the biological sample is blood, serum, or plasma.

5. The method according to claim 1, wherein the immune-mediated inflammatory disease **characterized by** an increase in expression of MMP12 is Takayasu arteritis.

6. The method according to claim 1, further comprising bringing the biological sample obtained from the subject into contact with a peptide having a site that can be cleaved by MMP12, wherein
the measuring the level of the MMP12 protein includes measuring a change caused by cleavage of the peptide having a site that can be cleaved by MMP12.

7. The method according to any one of claims 1 to 5, wherein
the method is a method for predicting a possibility of developing the immune-mediated inflammatory disease **characterized by** an increase in expression of MMP12, in a subject,
the method further comprises comparing the level of the MMP12 protein in the biological sample obtained from the subject with a reference value, and
if the level of the MMP12 protein in the biological sample obtained from the subject is higher than the reference value, it indicates that there is a high possibility that the subject will develop the immune-mediated inflammatory disease.

8. The method according to any one of claims 1 to 5, wherein
the method is a method for predicting prognosis of a subject with the immune-mediated inflammatory disease **characterized by** an increase in expression of MMP12,
the subject is a subject who has received treatment for the immune-mediated inflammatory disease **characterized by** an increase in expression of MMP12,
the method further comprises comparing the level of the MMP12 protein in the biological sample obtained from the subject with a reference value, and
if the level of the MMP12 protein in the biological sample obtained from the subject is higher than the reference value, it indicates that there is a high possibility of relapse of the immune-mediated inflammatory disease **characterized by** an increase in expression of MMP12, in the subject.

9. The method according to any one of claims 1 to 5, wherein
the method is a method for predicting a post-treatment condition of a subject with the immune-mediated inflammatory disease **characterized by** an increase in expression of MMP12,
the subject is a subject who has received treatment for the immune-mediated inflammatory disease **characterized by** an increase in expression of MMP12,
the method further comprises comparing the level of the MMP12 protein in the biological sample obtained from the subject with a reference value, and
if the level of the MMP12 protein in the biological sample obtained from the subject is lower than the reference value, it indicates that there is a high possibility that the immune-mediated inflammatory disease **characterized by** an increase in expression of MMP12, in the subject, has remitted.

10. The method according to any one of claims 1 to 5, wherein
the method is a method for evaluating timing of administration of a therapeutic drug for the immune-mediated inflammatory disease **characterized by** an increase in expression of MMP12, to a subject with the immune-mediated inflammatory disease **characterized by** an increase in expression of MMP12,
the method further comprises comparing the level of the MMP12 protein in the biological sample obtained from the subject with a reference value, and
a fact that the level of the MMP12 protein in the biological sample obtained from the subject is lower than the reference value is an indicator for discontinuing the administration of the drug to the subject.

11. A diagnostic biomarker for an immune-mediated inflammatory disease **characterized by** an increase in expression of MMP12, comprising MMP12.

12. A diagnostic drug for an immune-mediated inflammatory disease **characterized by** an increase in expression of MMP12, containing a substance that specifically interacts with MMP12.

13. The diagnostic drug according to claim 12, wherein the diagnostic drug is a diagnostic drug including a conjugate of a peptide having a site that can be cleaved by MMP12 and a label substance.

14. The diagnostic drug according to claim 12, wherein the conjugate includes a conjugate including a peptide having a site that can be cleaved by MMP12, a fluorophore bound to one end of the peptide, and a quencher bound to another end of the peptide.

15. The diagnostic drug according to any one of claims 12 to 14, wherein the immune-mediated inflammatory disease **characterized by** an increase in expression of MMP12 is selected from the group consisting of vasculitis syndrome, granulomatous vasculitis, IgG4 related disease, inflammatory bowel disease, sarcoidosis, tuberculous mycobacterial infection, and nontuberculous mycobacterial infection.

16. A diagnostic kit for diagnosing an immune-mediated inflammatory disease **characterized by** an increase in expression of MMP12, the diagnostic kit comprising a substance that specifically interacts with an MMP12 protein.

17. The diagnostic kit according to claim 16, wherein the substance that specifically interacts with the MMP12 protein includes an antibody to MMP12, an antigen-binding fragment of an antibody to MMP12, an aptamer to MMP12, or a peptide having a site that can be cleaved by MMP12.

18. A method for screening for a substance effective for treatment for an immune-mediated inflammatory disease **characterized by** an increase in expression of MMP12, the method comprising:
measuring a level of an MMP12 protein in a biological sample obtained from a subject after administration of a test substance; and
if the level of the MMP12 protein is decreased due to the administration of the test substance, selecting the test substance as a candidate substance effective for treatment for the immune-mediated inflammatory disease **characterized by** an increase in expression of MMP12.

19. A therapeutic agent for an immune-mediated inflammatory disease **characterized by** an increase in expression of MMP12, containing an MMP12 inhibitory substance.
